(19) Europäisches Patentamt / European Patent Office / Office européen des brevets

(11) **EP 4 061 813 B1**

(12) # EUROPEAN PATENT SPECIFICATION

(45) Date of publication and mention of the grant of the patent: **04.06.2025 Bulletin 2025/23**

(21) Application number: **20807432.8**

(22) Date of filing: **20.11.2020**

(51) International Patent Classification (IPC): ***C07D 471/04*** *(2006.01)* ***A61P 25/00*** *(2006.01)* ***A61K 31/401*** *(2006.01)*

(52) Cooperative Patent Classification (CPC): **A61P 25/00; C07D 471/04**

(86) International application number: **PCT/EP2020/082802**

(87) International publication number: **WO 2021/099527 (27.05.2021 Gazette 2021/21)**

(54) **PYRROLIDINE DERIVATIVES**

PYRROLIDINDERIVATE

DÉRIVÉS DE PYRROLIDINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.11.2019 EP 19210948**

(43) Date of publication of application: **28.09.2022 Bulletin 2022/39**

(73) Proprietor: **F. Hoffmann-La Roche AG**
**4070 Basel (CH)**

(72) Inventors:
- **KRAMER, Christian**
  **4070 Basel (CH)**
- **PINARD, Emmanuel**
  **4070 Basel (CH)**
- **RATNI, Hasane**
  **4070 Basel (CH)**

(74) Representative: **Belkacemi, Doria**
**F. Hoffmann-La Roche AG**
**Grenzacherstrasse 124**
**4070 Basel (CH)**

(56) References cited:
**WO-A1-2018/002760 WO-A1-2018/069732**

- **SCHECHTMANN G ET AL: "Cholinergic mechanisms involved in the pain relieving effect of spinal cord stimulation in a model of neuropathy", PAIN, ELSEVIER SCIENCE PUBLISHERS, AMSTERDAM, NL, vol. 139, no. 1, 30 September 2008 (2008-09-30), pages 136 - 145, XP025430377, ISSN: 0304-3959, [retrieved on 20080509], DOI: 10.1016/J.PAIN.2008.03.023**
- **SHEN WEIXING ET AL: "M4 Muscarinic Receptor Signaling Ameliorates Striatal Plasticity Deficits in Models of L-DOPA-Induced Dyskinesia", NEURON, CELL PRESS, US, vol. 88, no. 4, 18 November 2015 (2015-11-18), pages 762 - 773, XP029303595, ISSN: 0896-6273, DOI: 10.1016/J.NEURON.2015.10.039**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention relates to pyrrolidine derivatives useful as M4 positive allosteric modulators, their manufacture, pharmaceutical compositions comprising said derivatives and their use as medicaments for the therapeutic and/or prophylactic treatment of M4-mediated diseases or disorders, such as Alzheimer's disease, schizophrenia, psychosis, Parkinson's disease, pain, addiction and Huntington's disease.

### Background of the invention

Muscarinic Acetylcholine receptors are G-protein-coupled receptors (GPCRs) and consist of five subtypes termed M1, M2, M3, M4 and M5. The M4 receptor is coupled to Gi and is mainly expressed in the cerebral cortex, striatum, hypothalamus and hippocampus (Lebois, et al., Neuropharmacology 2018, 136, 362-373).

A genome wide association study of schizophrenia (11,260 cases and 24,542 controls) identified a single nucleotide polymorphism significantly linked to disease at locus rs7951870, which includes the M4 gene (Pardiñas, et al., Nature genetics 2018, 50, 381-389).

Cholinergic neurotransmission is known to have an important role in cognitive functions as evidenced by the fact that cholinergic receptor antagonists cause profound memory impairments and acetylcholinesterase inhibitors like donepezil show pro-cognitive effects in Alzheimer's disease. In schizophrenia, a hyper-dopaminergic state in the striatum and nucleus accumbens has been associated with psychosis and is the target of current antipsychotic drugs blocking the dopamine D2 receptor.

Recent development of M4 specific positive allosteric modulators (PAMs), that potentiate the effects of the endogenous agonist acetylcholine, has uncovered a role for these receptors in the control of dopamine release in the striatum and in key synapses known to be important for cognition in the hippocampus.

In a recent study, it was demonstrated that M4 PAMs reduce striatal dopamine release after amphetamine treatment in wild-type mice but not M4 knock-outs (Byun, et al., Neuropsychopharmacology 2014, 39, 1578). Another study showed that M4 PAM induced inhibition of glutamate excitatory synaptic transmission at the Schaeffer collateral - CA1 synapse in the hippocampus (Thorn, et al., Hippocampus 2017, 27, 794-810).

Further rodent in vivo studies showed that the M4 PAM VU0467154 ameliorated the associative learning impairments in the touch screen pair-wise visual discrimination task induced by MK-801, a non-competitive NMDA receptor antagonist. These effects were absent in the M4 knockout mice, demonstrating the specificity of this phenotype to the M4 receptor (Bubser, et al., ACS Chemical Neuroscience 2014, 5, 920-942).

Activating the striatal and hippocampal M4 receptors by using a specific PAM can reduce the hyperdopaminergic state in the striatum and the excessive stimulation of the hippocampus, providing a treatment of psychosis and cognitive impairment in schizophrenia.

Activation of muscarinic receptors by the M1/M4 agonist Xanomeline has been shown to improve cognition and psychiatric symptoms like hallucinations, delusions and vocal outbursts in a clinical study with 345 Alzheimer patients (Bodick, et al., Arch Neurol 1997, 54, 465-73). An M4 PAM could therefore improve cognitive deficiencies in Alzheimer patients and reduce psychiatric symptoms.

M4 receptors are known to control dopamine release in the striatum (Tzavara, et al., The FASEB Journal 2004, 18, 1410-1412), a region important for reward and addiction. It was shown that M4 knock-out mice have higher cocaine self-administration in an operant behaviour test (Schmidt, et al., Psychopharmacology 2011, 216, 367-378) and conversely that a M4 PAM tool compound is reducing cocaine self-administration in mice (Dencker, et al., Psychopharmacology 2012, 224, 277-287). These results indicate that activation of the M4 receptor may reduce addiction.

The disease is caused by a triplet repeat expansion coding for Glutamine in the Huntington gene, which cause neurodegeneration, leading to movement abnormalities, cognitive impairments evolving to dementia and death.

It was shown that chronic treatment with a M4 PAM can improve the motor and synaptic defects in a mouse model of Huntington (YAC128 mice) (Pancani, et al., Proceedings of the National Academy of Sciences 2015, 112, 14078-14083). An M4 PAM may normalize early changes in corticostriatal transmission and reduce the progression of Huntington's disease.

Parkinson's disease is caused by a degeneration of dopamine producing neurons in the substantia nigra. The lack of dopamine is causing movement disorders that can be treated for a certain period by L-DOPA supplementation. This treatment losses its efficacy overtime and higher doses of L-DOPA are needed to control the symptoms, higher doses are also inducing dyskinesia. It was shown in a mouse 6-hydroxydopamine lesion model of Parkinson, that M4 PAM treatment can reduce the L-DOPA induced dyskinesia, these findings were reproduced in a monkey MPTP model of Parkinson (Shen, et al., Neuron 2015, 88, 762-773). An M4 PAM could therefore be used as a symptomatic treatment of L-DOPA induced dyskinesia in Parkinson's disease.

An M4 antagonist was shown to block the antinociceptive effect of electrical spinal cord stimulation in a rat neuropathic pain model (Schechtmann, et al., Pain 2008, 139, 136-145), also M4 knock-down in the rat spinal cord has been shown to increase heat nociception in rats (Cai, et al., Journal of Neurochemistry 2009, 111, 1000-1010). Therefore, M4 activation could reduce pain sensation.

WO 2018/069732 A1 discloses heterocyclic compounds useful as agonists of the muscarinic M1 receptor or M1 and M4 receptors.

Therefore, modulating the M4 receptor activity is a promising therapeutic strategy for the treatment or prevention of M4-mediated diseases or disorders, such as Alzheimer's disease, schizophrenia, psychosis, Parkinson's disease, pain, addiction and Huntington's disease.

There is a need for new compounds, formulations, treatments and therapies to treat or prevent M4-mediated diseases or disorders. It is, therefore, an object of this invention to provide compounds useful for the treatment or prevention or amelioration of such diseases and disorders with improved therapeutic properties, in particular improved pharmacokinetic properties.

## Summary of the invention

The present disclosure relates to a compound of formula (I)

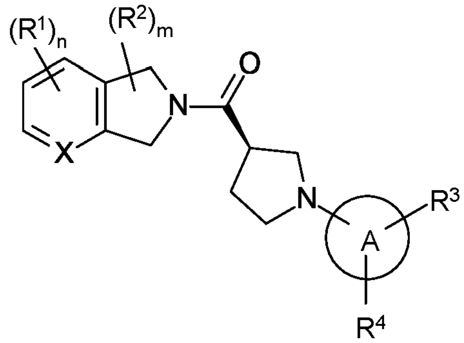

(I)

or a pharmaceutically acceptable salt thereof, wherein:

- A is phenyl or 6-membered heteroaryl group;
- X is $CR^8$ or N;
- $R^1$ is $C_1$-$C_6$-alkyl, $C_1$-$C_6$-haloalkyl, $C_1$-$C_6$-alkoxy, $C_1$-$C_6$-haloalkoxy, $C_2$-$C_8$-alkoxyalkyl, halogen or cyano, and $R^1$ may be different if n is 2 or 3;
- $R^2$ is $C_1$-$C_6$-alkyl or hydroxy, and $R^2$ may be different if m is 2;
- $R^3$ and $R^4$ are each independently selected from hydrogen, $C_1$-$C_6$-alkyl, $C_1$-$C_6$-haloalkyl, $C_1$-$C_6$-alkoxy, $C_1$-$C_6$-haloalkoxy, halogen, cyano, hydroxy, $C_3$-$C_{12}$-cycloalkyl, $C_2$-$C_9$-heterocycloalkyl, $-NR^5R^6$ and $-S(O)_2-R^7$;
- $R^5$, $R^6$ and $R^7$ are each independently selected from hydrogen and $C_1$-$C_6$-alkyl;
- $R^8$ is hydrogen, $C_1$-$C_6$-alkyl, $C_1$-$C_6$-haloalkyl, $C_1$-$C_6$-alkoxy, $C_1$-$C_6$-haloalkoxy, $C_2$-$C_8$-alkoxyalkyl, halogen or cyano, and $R^1$ may be different if n is 2 or 3;
- n is 0, 1, 2 or 3; and
- m is 0, 1 or 2.

A first object of the invention is a compound of formula (I)

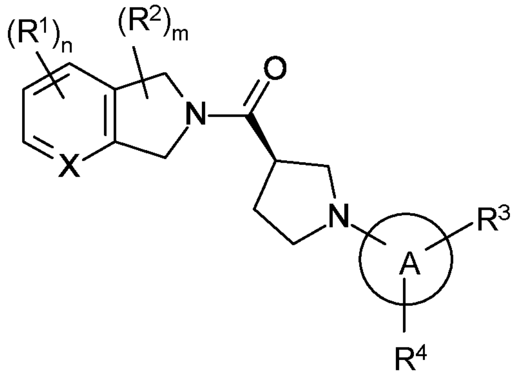

(I)

or a pharmaceutically acceptable salt thereof, wherein:

A is a 6-membered heteroaryl group selected from pyridinyl, pyradizinyl, pyrazinyl and pyrimidinyl;
$R^1$ is halogen, $C_1$-$C_6$-alkyl or $C_2$-$C_8$-alkoxyalkyl;
$R^3$ and $R^4$ are each independently selected from hydrogen, $C_1$-$C_6$-alkyl, $C_1$-$C_6$-haloalkyl, $C_1$-$C_6$-alkoxy, $C_1$-$C_6$-haloalkoxy, cyano, $C_3$-$C_{12}$-cycloalkyl, $C_2$-$C_9$-heterocycloalkyl, -$NR^5R^6$ and - $S(O)_2$-$R^7$;
$R^5$ and $R^6$ are each independently selected from hydrogen and $C_1$-$C_6$-alkyl, and $R^7$ is $C_1$-$C_6$-alkyl;
X is N;
n is 3; and
m is 0.

A further object of the invention is a process for the preparation of a compound of formula (I) as described herein, or a pharmaceutically acceptable salt thereof, comprising reacting an amine salt 1

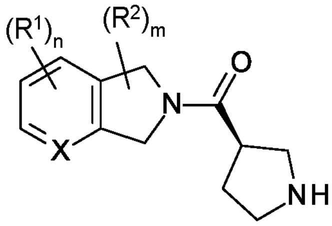

. 2HCl **1**

with a halide compound 2

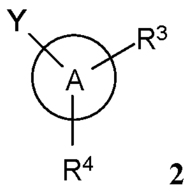

**2**

wherein $R^1$, $R^2$, $R^3$, $R^4$, X and A are as defined above, and Y is a halogen, to form said compound of formula (I), and optionally converting the compounds obtained into a pharmaceutically acceptable salt thereof.

The present disclosure provides a compound of formula (I) as described herein, or a pharmaceutically acceptable salt thereof, when manufactured according to the process as described above.

A further object of the present invention is a compound as described herein, or a pharmaceutically acceptable salt thereof, for use as therapeutically active substance.

A further object of the present invention is a pharmaceutical composition comprising a compound of formula (I) as described herein, or a pharmaceutically acceptable salt thereof, and a pharmaceutically acceptable excipient.

A further object of the invention is a compound of formula (I) as described herein, or a pharmaceutically acceptable salt thereof, for use in the therapeutic and/or prophylactic treatment of Alzheimer's disease, schizophrenia, psychosis, Parkinson's disease, pain, addiction and Huntington's disease.

The present disclosure provides the use of a compound of formula (I) as described herein, or a pharmaceutically

acceptable salt thereof, for the therapeutic and/or prophylactic treatment of Alzheimer's disease, schizophrenia, psychosis, Parkinson's disease, pain, addiction and Huntington's disease.

The present disclosure provides the use of a compound of formula (I) as described herein, or a pharmaceutically acceptable salt thereof, for the preparation of a medicament for the therapeutic and/or prophylactic treatment of Alzheimer's disease, schizophrenia, psychosis, Parkinson's disease, pain, addiction and Huntington's disease.

The present disclosure provides a method for the therapeutic and/or prophylactic treatment of Alzheimer's disease, schizophrenia, psychosis, Parkinson's disease, pain, addiction and Huntington's disease, which method comprises administering an effective amount of a compound of formula (I) as described herein, or a pharmaceutically acceptable salt thereof.

It has been surprisingly found that compounds of the present invention have a M4 positive allosteric modulation activity with improved pharmacokinetic properties, particularly a reduced clearance.

## Detailed description of the invention

The following definitions of the general terms used in the present description apply irrespectively of whether the terms in question appear alone or in combination with other groups.

The term "alkyl" refers to a mono- or multivalent, e.g., a mono- or bivalent, linear or branched saturated hydrocarbon group of 1 to 6 carbon atoms ("$C_1$-$C_6$-alkyl"), e.g., 1, 2, 3, 4, 5, or 6 carbon atoms. In some embodiments, the alkyl group contains 1 to 3 carbon atoms, e.g., 1, 2 or 3 carbon atoms. Some non-limiting examples of alkyl include methyl (Me), ethyl (Et), propyl, 2-propyl (isopropyl), n-butyl, iso-butyl, sec-butyl, tert-butyl, and 2,2-dimethylpropyl. Particular alkyl groups have 1 to 4 carbon atoms ("$C_{1-4}$-alkyl"). A particularly preferred, yet non-limiting example of alkyl is methyl.

The term "alkoxy" refers to an alkyl group, as previously defined, attached to the parent molecular moiety via an oxygen atom. Unless otherwise specified, the alkoxy group contains 1 to 6 carbon atoms ("$C_1$-$C_6$-alkoxy"). In some preferred embodiments, the alkoxy group contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "alkoxyalkyl" refers to an alkyl group, wherein at least one of the hydrogen atoms of the alkyl group has been replaced by an alkoxy group. Preferably, "alkoxyalkyl" refers to an alkyl group wherein 1, 2 or 3 hydrogen atoms, most preferably one hydrogen atom of the alkyl group have been replaced by an alkoxy group. Particularly preferred, yet non-limiting examples of alkoxyalkyl is methoxymethyl and 2-methoxyethyl.

The term "aryl" refers to a monocyclic, bicyclic, or tricyclic carbocyclic ring system having a total of 6 to 14 ring members ("$C_6$-$C_{14}$-aryl"), preferably, 6 to 12 ring members, and more preferably 6 to 10 ring members, and wherein at least one ring in the system is aromatic. A particularly preferred, yet non-limiting example of aryl is phenyl.

The terms "asymmetric carbon atom" and "asymmetric center" mean a carbon atom with four different substituents. According to the Cahn-Ingold-Prelog Convention, an asymmetric carbon atom can be of the "R" or "S" configuration.

The term "cyano" refers to a -CN (nitrile) group.

The term "cycloalkyl" as used herein refers to a saturated or partly unsaturated monocyclic or bicyclic hydrocarbon group of 3 to 12 ring carbon atoms ("$C_3$-$C_{12}$-cycloalkyl"). In some preferred embodiments, the cycloalkyl group is a saturated monocyclic hydrocarbon group of 3 to 10 ring carbon atoms, in particular 3 to 8 ring carbon atoms. "Bicyclic cycloalkyl" refers to cycloalkyl moieties consisting of two saturated carbocycles having two carbon atoms in common, i.e., the bridge separating the two rings is either a single bond or a chain of one or two ring atoms, and to spirocyclic moieties, i.e., the two rings are connected via one common ring atom. Preferably, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl, cycloheptyl, and spiro[2.3]hexan-5-yl.

The terms "halogen" or "halo", alone or in combination, denotes fluoro, chloro, bromo or iodo and particularly fluoro, chloro or bromo, more particularly fluoro and chloro. The term "halo", in combination with another group, denotes the substitution of said group with at least one halogen, particularly substituted with one to five halogens, particularly one to four halogens, i.e. one, two, three or four halogens.

The term "haloalkyl" refers to an alkyl group, as previously defined, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. Particularly preferred, yet non-limiting examples of haloalkyl are trifluoromethyl and trifluoroethyl.

The term "haloalkoxy" refers to an alkoxy group, wherein at least one of the hydrogen atoms of the alkoxy group has been replaced by a halogen atom, preferably fluoro. Preferably, "haloalkoxy" refers to an alkoxy group wherein 1, 2 or 3 hydrogen atoms of the alkoxy group have been replaced by a halogen atom, most preferably fluoro. Particularly preferred, yet non-limiting examples of haloalkoxy are difluoromethoxy and trifluoromethoxy.

The term "heteroaryl" refers to a mono- or multivalent, monocyclic or bicyclic, preferably bicyclic ring system having a total of 5 to 14 ring members, preferably, 5 to 12 ring members, and more preferably 5 to 10 ring members, wherein at least one ring in the system is aromatic, and at least one ring in the system contains one or more heteroatoms. Preferably, "heteroaryl" refers to a 5-10 membered heteroaryl comprising 1, 2, 3 or 4 heteroatoms independently selected from O, S and N. Most preferably, "heteroaryl" refers to a 5-10 membered heteroaryl comprising 1 to 2 heteroatoms independently selected from O and N. Some non-limiting examples of heteroaryl include 2-pyridyl, 3-pyridyl, 4-pyridyl, indol-1-yl, 1H-indol-2-yl, 1H-indol-3-yl, 1H-indol-4-yl, 1H-indol-5-yl, 1H-indol-6-yl, 1H-indol-7-yl, 1,2-benzoxazol-3-yl, 1,2-benzoxazol-4-yl, 1,2-benzoxazol-5-yl, 1,2-benzoxazol-6-yl, 1,2-benzoxazol-7-yl, 1H-indazol-3-yl, 1H-indazol-4-yl, 1H-indazol-5-yl, 1H-indazol-6-yl, 1H-indazol-7-yl, pyrazol-1-yl, 1H-pyrazol-3-yl, 1H-pyrazol-4-yl, 1H-pyrazol-5-yl, imidazol-1-yl, 1H-imidazol-2-yl, 1H-imidazol-4-yl, 1H-imidazol-5-yl, oxazol-2-yl, oxazol-4-yl, oxazol-5-yl, thiazol-4-yl, and 1,2,4-oxadiazol-3-yl. Most preferably, "heteroaryl" refers to 3-pyridyl, 4-pyridyl, 1H-pyrazol-5-yl, thiazol-4-yl, or 1,2,4-oxadiazol-3-yl.

The term "heterocycloalkyl" refers to a saturated or partly unsaturated mono- or bicyclic, preferably monocyclic ring system of 3 to 10 ring atoms, preferably 3 to 8 ring atoms, wherein 1, 2, or 3 of said ring atoms are heteroatoms selected from N, O and S, the remaining ring atoms being carbon. Preferably, 1 to 2 of said ring atoms are selected from N and O, the remaining ring atoms being carbon. "Bicyclic heterocyclyl" refers to heterocyclic moieties consisting of two cycles having two ring atoms in common, i.e., the bridge separating the two rings is either a single bond or a chain of one or two ring atoms, and to spirocyclic moieties, i.e., the two rings are connected via one common ring atom. Some non-limiting examples of heterocyclyl groups include azetidin-3-yl, azetidin-2-yl, oxetan-3-yl, oxetan-2-yl, 2-oxopyrrolidin-1-yl, 2-oxopyrrolidin-3-yl, 5-oxopyrrolidin-2-yl, 5-oxopyrrolidin-3-yl, 2-oxo-1-piperidyl, 2-oxo-3-piperidyl, 2-oxo-4-piperidyl, 6-oxo-2-piperidyl, 6-oxo-3-piperidyl, 1-piperidinyl, 2-piperidinyl, 3-piperidinyl, 4-piperidinyl, morpholino, morpholin-2-yl, morpholin-3-yl, pyrrolidinyl (e.g., pyrrolidin-3-yl), 3-azabicyclo[3.1.0]hexan-6-yl, or 2,5-diazabicyclo[2.2.1]heptan-2-yl.

The term "hydroxy" refers to an -OH group.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein.

Particularly preferred pharmaceutically acceptable salts of compounds of formula (I) are the salts of hydrochloric acid, hydrobromic acid, sulfuric acid, phosphoric acid and methanesulfonic acid.

The term "protecting group" (PG) denotes the group which selectively blocks a reactive site in a multifunctional compound such that a chemical reaction can be carried out selectively at another unprotected reactive site in the meaning conventionally associated with it in synthetic chemistry. Protective groups can be removed at the appropriate point. Exemplary protective groups are amino-protective groups, carboxy-protective groups or hydroxy-protective groups. Particular protective groups are the tert-butoxycarbonyl (Boc), benzyloxycarbonyl (Cbz), fluorenylmethoxycarbonyl (Fmoc) and benzyl (Bn). Further particular protective groups are the tert-butoxycarbonyl (Boc) and the fluorenylmethoxycarbonyl (Fmoc). More particular protective group is the tert-butoxycarbonyl (Boc). Exemplary protective groups and their application in organic synthesis are described, for example, in "Protective Groups in Organic Chemistry" by T. W. Greene and P. G. M. Wutts, 5th Ed., 2014, John Wiley & Sons, N.Y.

The term "$EC_x$" is the half maximal effective concentration and denotes the plasma concentration of a particular compound required for obtaining x% of the maximum of a particular effect in vivo. Examples of "$EC_x$" are $EC_{20}$, $EC_{50}$ and $EC_{100}$ denoting the plasma concentration of a particular compound required for obtaining 20%, 50% and 100%, respectively, of the maximum of a particular effect in vivo.

The term "treatment" as used herein includes: (1) inhibiting the state, disease, disorder or condition (e.g. arresting, reducing or delaying the development of the disease, or a relapse thereof in case of maintenance treatment, of at least one clinical or subclinical symptom thereof); and/or (2) relieving the condition or disease (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The following abbreviations are used in the present text:

BOC = tert-butyloxycarbonyl, BSA = bovine serum albumin, CAS RN = chemical abstracts registration number, cAMP = cyclic adenosine monophosphate, DMA = dimethylacetamide,

DMEM = Dulbecco's modified eagle media, DMF = dimethylformamide, DMSO = dimethylsulfoxide, EtOAc = ethyl acetate, FBS = fetal bovine serum, FMO = flavin-containing monooxygenase, FRET = Förster resonance energy transfer, h = hour(s), Hal = halogen, HBSS = Hank's balanced salt solution, HEPES = 4-(2-hydroxyethyl)-1-piperazineethanesulfonic acid,

HPLC = high-performance liquid chromatography, IBMX = 3-isobutyl-1-methylxanthine,
$iPr_2Net$ = N, N-diisopropylethylamine, MeOH = methanol, min = minute(s), ml = milliliter, μl = microliter, MS = mass spectrum, NaOH = sodium hydroxide, $Na_2SO_4$ = sodium sulfate,
NaOtBu= sodium tert-butyloxide, $Pd_2(dba)_3$ = tris(dibenzylideneacetone)dipalladium(0), PAM = positive allosteric modulator, PBS = phosphate-buffered saline, R = any group, RT = room temperature, TBTU = 2-(1H-Benzotriazole-1-yl)-1,1,3,3-tetramethylaminium tetrafluoroborate,
tBuXPhos = 2-di-tert-butylphosphino-2',4',6'-triisopropylbiphenyl, THF = tetrahydrofuran, UGT = UDP-glucuronosyltransferase.

Compounds of the invention

The present disclosure provides a compound of formula (I)

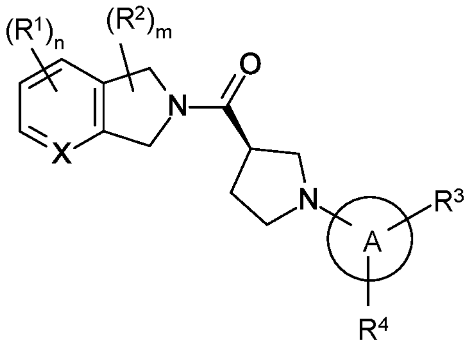

(I)

or a pharmaceutically acceptable salt thereof, wherein:

A is phenyl or 6-membered heteroaryl group;
X is $CR^8$ or N;
$R^1$ is $C_1$-$C_6$-alkyl, $C_1$-$C_6$-haloalkyl, $C_1$-$C_6$-alkoxy, $C_1$-$C_6$-haloalkoxy, $C_2$-$C_8$-alkoxyalkyl, halogen or cyano, and $R^1$ may be different if n is 2 or 3;
$R^2$ is $C_1$-$C_6$-alkyl or hydroxy, and $R^2$ may be different if m is 2;
$R^3$ and $R^4$ are each independently selected from hydrogen, $C_1$-$C_6$-alkyl, $C_1$-$C_6$-haloalkyl, $C_1$-$C_6$-alkoxy, $C_1$-$C_6$-haloalkoxy, halogen, cyano, hydroxy, $C_3$-$C_{12}$-cycloalkyl, $C_2$-$C_9$-heterocycloalkyl, $-NR^5R^6$ and $-S(O)_2-R^7$;
$R^5$, $R^6$ and $R^7$ are each independently selected from hydrogen and $C_1$-$C_6$-alkyl;
$R^8$ is hydrogen, $C_1$-$C_6$-alkyl, $C_1$-$C_6$-haloalkyl, $C_1$-$C_6$-alkoxy, $C_1$-$C_6$-haloalkoxy, $C_2$-$C_8$-alkoxyalkyl, halogen or cyano, and $R^1$ may be different if n is 2 or 3;
n is 0, 1, 2 or 3; and
m is 0, 1 or 2.

The present invention provides a compound of formula (I)

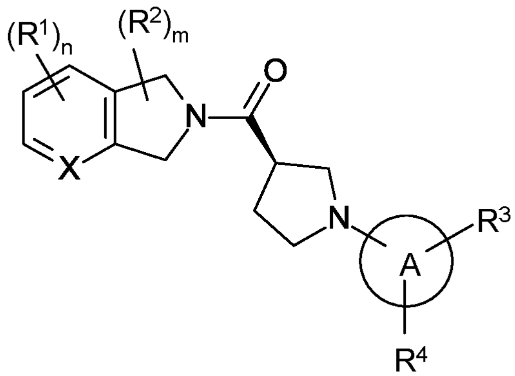

(I)

or a pharmaceutically acceptable salt thereof, wherein:

A is a 6-membered heteroaryl group selected from pyridinyl, pyradizinyl, pyrazinyl and pyrimidinyl;
$R^1$ is halogen, $C_1$-$C_6$-alkyl or $C_2$-$C_8$-alkoxyalkyl;
$R^3$ and $R^4$ are each independently selected from hydrogen, $C_1$-$C_6$-alkyl, $C_1$-$C_6$-haloalkyl, $C_1$-$C_6$-alkoxy, $C_1$-$C_6$-haloalkoxy, cyano, $C_3$-$C_{12}$-cycloalkyl, $C_2$-$C_9$-heterocycloalkyl, -$NR^5R^6$ and - $S(O)_2$-$R^7$;
$R^5$ and $R^6$ are each independently selected from hydrogen and $C_1$-$C_6$-alkyl, and $R^7$ is $C_1$-$C_6$-alkyl;
X is N;
n is 3; and
m is 0.

In one embodiment, the present disclosure provides a compound of formula (I) as described herein, wherein A is a 6-membered heteroaryl group selected from pyridinyl, pyradizinyl, pyrazinyl and pyrimidinyl.
In a preferred embodiment, there is provided a compound of formula (I) as described herein, or a pharmaceutically acceptable salt thereof, wherein A is pyridinyl or pyrimidinyl.
In a further embodiment, there is provided a compound of formula (I) as described herein, or a pharmaceutically acceptable salt thereof, wherein $R^1$ is halogen, $C_1$-$C_6$-alkyl or $C_2$-$C_8$-alkoxyalkyl.
In a preferred embodiment, there is provided a compound of formula (I) as described herein, or a pharmaceutically acceptable salt thereof, wherein $R^1$ is chlorine, methyl or methoxymethyl.
In a further embodiment, there is provided a compound of formula (I) as described herein, or a pharmaceutically acceptable salt thereof, wherein $R^3$ and $R^4$ are each independently selected from hydrogen, $C_1$-$C_6$-alkyl, $C_1$-$C_6$-haloalkyl, $C_1$-$C_6$-alkoxy, $C_1$-$C_6$-haloalkoxy, cyano, $C_3$-$C_{12}$-cycloalkyl, $C_2$-$C_9$-heterocycloalkyl, -$NR^5R^6$ and -$S(O)_2$-$R^7$, wherein $R^5$ and $R^6$ are each independently selected from hydrogen and $C_1$-$C_6$-alkyl, and $R^7$ is $C_1$-$C_6$-alkyl.
In a preferred embodiment, there is provided a compound of formula (I) as described herein, or a pharmaceutically acceptable salt thereof, wherein $R^3$ and $R^4$ are each independently selected from hydrogen, $C_1$-$C_6$-haloalkyl, $C_1$-$C_6$-haloalkoxy and cyano.
In a more preferred embodiment, there is provided a compound of formula (I) as described herein, or a pharmaceutically acceptable salt thereof, wherein $R^3$ and $R^4$ are each independently selected from hydrogen, $-CHF_2$, $-CF_3$, $-OCHF_2$ and cyano.
In a further embodiment, there is provided a compound of formula (I) as described herein, or a pharmaceutically acceptable salt thereof, wherein X is N.
In a further embodiment, there is provided a compound of formula (I) as described herein, or a pharmaceutically acceptable salt thereof, wherein n is 3.
In a further embodiment, there is provided a compound of formula (I) as described herein, or a pharmaceutically acceptable salt thereof, wherein m is 0.
In one embodiment, there is provided a compound of formula (I) as described herein, or a pharmaceutically acceptable salt thereof, wherein:

A is a 6-membered heteroaryl group selected from pyridinyl, pyradizinyl, pyrazinyl and pyrimidinyl;
$R^1$ is halogen, $C_1$-$C_6$-alkyl or $C_2$-$C_8$-alkoxyalkyl;
$R^3$ and $R^4$ are each independently selected from hydrogen, $C_1$-$C_6$-alkyl, $C_1$-$C_6$-haloalkyl, $C_1$-$C_6$-alkoxy, $C_1$-$C_6$-haloalkoxy, cyano, $C_3$-$C_{12}$-cycloalkyl, $C_2$-$C_9$-heterocycloalkyl, -$NR^5R^6$ and - $S(O)_2$-$R^7$;
$R^5$ and $R^6$ are each independently selected from hydrogen and $C_1$-$C_6$-alkyl, and $R^7$ is $C_1$-$C_6$-alkyl;
X is N;
n is 3; and
m is 0.

In a preferred embodiment, there is provided a compound of formula (I) as described herein, or a pharmaceutically acceptable salt thereof, wherein:

A is pyridinyl or pyrimidinyl;
$R^1$ is halogen, $C_1$-$C_6$-alkyl or $C_2$-$C_8$-alkoxyalkyl;
$R^3$ and $R^4$ are each independently selected from hydrogen, $C_1$-$C_6$-haloalkyl, $C_1$-$C_6$-haloalkoxy and cyano;
X is N;
n is 3; and
m is 0.

In a more preferred embodiment, there is provided a compound of formula (I) as described herein, or a pharmaceutically acceptable salt thereof, wherein:

A is pyridinyl or pyrimidinyl;
$R^1$ is chlorine, methyl or methoxymethyl;
$R^3$ and $R^4$ are each independently selected from hydrogen, $-CHF_2$, $-CF_3$, $-OCHF_2$ and cyano;
X is N;
n is 3; and
m is 0.

The compounds of formula (I), or pharmaceutically acceptable salts thereof, may contain one or more asymmetric centers and can be present in the form of optically pure enantiomers, mixtures of enantiomers such as, for example, racemates, mixtures of diastereoisomers, diastereoisomeric racemates or mixtures of diastereoisomeric racemates.

In a further embodiment, there is provided a compound of formula (I) as described herein, or a pharmaceutically acceptable salt thereof, selected from:

(3-chloro-2,4-dimethyl-5,7-dihydropyrrolo[3,4-b]pyridin-6-yl)-[(3R)-1-(3-pyridyl)pyrrolidin-3-yl]methanone;
(3-chloro-2,4-dimethyl-5,7-dihydropyrrolo[3,4-b]pyridin-6-yl)-[(3R)-1-pyrimidin-4-ylpyrrolidin-3-yl]methanone;
(3-chloro-2,4-dimethyl-5,7-dihydropyrrolo[3,4-b]pyridin-6-yl)-[(3R)-1-(4-pyridyl)pyrrolidin-3-yl]methanone;
(3-chloro-2,4-dimethyl-5,7-dihydropyrrolo[3,4-b]pyridin-6-yl)-[(3R)-1-(6-methyl-3-pyridyl)pyrrolidin-3-yl]methanone;
(3-chloro-2,4-dimethyl-5,7-dihydropyrrolo[3,4-b]pyridin-6-yl)-[(3R)-1-(2-methyl-4-pyridyl)pyrrolidin-3-yl]methanone;
(3-chloro-2,4-dimethyl-5,7-dihydropyrrolo[3,4-b]pyridin-6-yl)-[(3R)-1-(2-cyclopropylpyrimidin-4-yl)pyrrolidin-3-yl]methanone;
(3-chloro-2,4-dimethyl-5,7-dihydropyrrolo[3,4-b]pyridin-6-yl)-[(3R)-1-[5-(trifluoromethyl)pyrimidin-2-yl]pyrrolidin-3-yl]methanone;
(3-chloro-2,4-dimethyl-5,7-dihydropyrrolo[3,4-b]pyridin-6-yl)-[(3R)-1-(5-methylpyrimidin-2-yl)pyrrolidin-3-yl]methanone;
(3-chloro-2,4-dimethyl-5,7-dihydropyrrolo[3,4-b]pyridin-6-yl)-[(3R)-1-[6-(trifluoromethyl)-3-pyridyl]pyrrolidin-3-yl]methanone;
(3-chloro-2,4-dimethyl-5,7-dihydropyrrolo[3,4-b]pyridin-6-yl)-[(3R)-1-(6-methoxy-3-pyridyl)pyrrolidin-3-yl]methanone;
(3-chloro-2,4-dimethyl-5,7-dihydropyrrolo[3,4-b]pyridin-6-yl)-[(3R)-1-[6-(methylamino)-3-pyridyl]pyrrolidin-3-yl]methanone;
(3-chloro-2,4-dimethyl-5,7-dihydropyrrolo[3,4-b]pyridin-6-yl)-[(3R)-1-(2-methyl-3-pyridyl)pyrrolidin-3-yl]methanone;
(3-chloro-2,4-dimethyl-5,7-dihydropyrrolo[3,4-b]pyridin-6-yl)-[(3R)-1-(4-methyl-3-pyridyl)pyrrolidin-3-yl]methanone;
(3-chloro-2,4-dimethyl-5,7-dihydropyrrolo[3,4-b]pyridin-6-yl)-[(3R)-1-[5-(trifluoromethyl)-3-pyridyl]pyrrolidin-3-yl]methanone;
(3-chloro-2,4-dimethyl-5,7-dihydropyrrolo[3,4-b]pyridin-6-yl)-[(3R)-1-[5-(difluoromethyl)-3-pyridyl]pyrrolidin-3-yl]methanone;
(3-chloro-2,4-dimethyl-5,7-dihydropyrrolo[3,4-b]pyridin-6-yl)-[(3R)-1-(5-cyclopropyl-3-pyridyl)pyrrolidin-3-yl]methanone;
(3-chloro-2,4-dimethyl-5,7-dihydropyrrolo[3,4-b]pyridin-6-yl)-[(3R)-1-[5-(difluoromethoxy)-3-pyridyl]pyrrolidin-3-yl]methanone;
(3-chloro-2,4-dimethyl-5,7-dihydropyrrolo[3,4-b]pyridin-6-yl)-[(3R)-1-(5-methoxy-3-pyridyl)pyrrolidin-3-yl]methanone;
(3-chloro-2,4-dimethyl-5,7-dihydropyrrolo[3,4-b]pyridin-6-yl)-[(3R)-1-(5-methylsulfonyl-3-pyridyl)pyrrolidin-3-yl]methanone;
(3-chloro-2,4-dimethyl-5,7-dihydropyrrolo[3,4-b]pyridin-6-yl)-[(3R)-1-[2-(difluoromethyl)-4-pyridyl]pyrrolidin-3-yl]methanone;
(3-chloro-2,4-dimethyl-5,7-dihydropyrrolo[3,4-b]pyridin-6-yl)-[(3R)-1-[2-(trifluoromethyl)-4-pyridyl]pyrrolidin-3-yl]methanone;
(3-chloro-2,4-dimethyl-5,7-dihydropyrrolo[3,4-b]pyridin-6-yl)-[(3R)-1-(2-methoxy-4-pyridyl)pyrrolidin-3-yl]methanone;
(3-chloro-2,4-dimethyl-5,7-dihydropyrrolo[3,4-b]pyridin-6-yl)-[(3R)-1-[2-(trifluoromethyl)pyrimidin-4-yl]pyrrolidin-3-yl]methanone;
4-[(3R)-3-(3-chloro-2,4-dimethyl-5,7-dihydropyrrolo[3,4-b]pyridine-6-carbonyl)pyrrolidin-1-yl]pyrimidine-2-carbonitrile;

(3-chloro-2,4-dimethyl-5,7-dihydropyrrolo[3,4-b]pyridin-6-yl)-[(3R)-1-(2-methoxypyrimidin-4-yl)pyrrolidin-3-yl]methanone;
(3-chloro-2,4-dimethyl-5,7-dihydropyrrolo[3,4-b]pyridin-6-yl)-[(3R)-1-(6-methylpyrimidin-4-yl)pyrrolidin-3-yl]methanone;
(3-chloro-2,4-dimethyl-5,7-dihydropyrrolo[3,4-b]pyridin-6-yl)-[(3R)-1-[6-(trifluoromethyl)pyrimidin-4-yl]pyrrolidin-3-yl]methanone;
(3-chloro-2,4-dimethyl-5,7-dihydropyrrolo[3,4-b]pyridin-6-yl)-[(3R)-1-(6-cyclopropylpyrimidin-4-yl)pyrrolidin-3-yl]methanone;
(3-chloro-2,4-dimethyl-5,7-dihydropyrrolo[3,4-b]pyridin-6-yl)-[(3R)-1-(6-methoxypyrimidin-4-yl)pyrrolidin-3-yl]methanone;
(3-chloro-2,4-dimethyl-5,7-dihydropyrrolo[3,4-b]pyridin-6-yl)-[(3R)-1-pyrimidin-5-ylpyrrolidin-3-yl]methanone;
(3-chloro-2,4-dimethyl-5,7-dihydropyrrolo[3,4-b]pyridin-6-yl)-[(3R)-1-(2-methylpyrimidin-5-yl)pyrrolidin-3-yl]methanone;
(3-chloro-2,4-dimethyl-5,7-dihydropyrrolo[3,4-b]pyridin-6-yl)-[(3R)-1-[2-(trifluoromethyl)pyrimidin-5-yl]pyrrolidin-3-yl]methanone;
(3-chloro-2,4-dimethyl-5,7-dihydropyrrolo[3,4-b]pyridin-6-yl)-[(3R)-1-(2-methoxypyrimidin-5-yl)pyrrolidin-3-yl]methanone;
(3-chloro-2,4-dimethyl-5,7-dihydropyrrolo[3,4-b]pyridin-6-yl)-[(3R)-1-[2-(oxetan-3-yl)pyrimidin-5-yl]pyrrolidin-3-yl]methanone;
(3-chloro-2,4-dimethyl-5,7-dihydropyrrolo[3,4-b]pyridin-6-yl)-[(3R)-1-[2-(methylamino)pyrimidin-5-yl]pyrrolidin-3-yl]methanone;
(3-chloro-2,4-dimethyl-5,7-dihydropyrrolo[3,4-b]pyridin-6-yl)-[(3R)-1-[2-(dimethylamino)pyrimidin-5-yl]pyrrolidin-3-yl]methanone;
(3-chloro-2,4-dimethyl-5,7-dihydropyrrolo[3,4-b]pyridin-6-yl)-[(3R)-1-(4-methylpyrimidin-5-yl)pyrrolidin-3-yl]methanone;
(3-chloro-2,4-dimethyl-5,7-dihydropyrrolo[3,4-b]pyridin-6-yl)-[(3R)-1-pyridazin-4-ylpyrrolidin-3-yl]methanone;
(3-chloro-2,4-dimethyl-5,7-dihydropyrrolo[3,4-b]pyridin-6-yl)-[(3R)-1-pyridazin-3-ylpyrrolidin-3-yl]methanone;
(3-chloro-2,4-dimethyl-5,7-dihydropyrrolo[3,4-b]pyridin-6-yl)-[(3R)-1-(6-methylpyridazin-3-yl)pyrrolidin-3-yl]methanone;
(3-chloro-2,4-dimethyl-5,7-dihydropyrrolo[3,4-b]pyridin-6-yl)-[(3R)-1-[6-(trifluoromethyl)pyridazin-3-yl]pyrrolidin-3-yl]methanone;
(3-chloro-2,4-dimethyl-5,7-dihydropyrrolo[3,4-b]pyridin-6-yl)-[(3R)-1-(6-methoxypyrazin-2-yl)pyrrolidin-3-yl]methanone;
(3-chloro-2,4-dimethyl-5,7-dihydropyrrolo[3,4-b]pyridin-6-yl)-[(3R)-1-(6-cyclopropylpyrazin-2-yl)pyrrolidin-3-yl]methanone;
5-[(3R)-3-(3-chloro-2,4-dimethyl-5,7-dihydropyrrolo[3,4-b]pyridine-6-carbonyl)pyrrolidin-1-yl]pyrazine-2-carbonitrile;
(3-chloro-2,4-dimethyl-5,7-dihydropyrrolo[3,4-b]pyridin-6-yl)-[(3R)-1-(5-methoxypyrazin-2-yl)pyrrolidin-3-yl]methanone;
(3-chloro-2,4-dimethyl-5,7-dihydropyrrolo[3,4-b]pyridin-6-yl)-[(3R)-1-(5-methylpyrazin-2-yl)pyrrolidin-3-yl]methanone;
(3-chloro-2,4-dimethyl-5,7-dihydropyrrolo[3,4-b]pyridin-6-yl)-[(3R)-1-[5-(trifluoromethyl)pyrazin-2-yl]pyrrolidin-3-yl]methanone;
(3-chloro-2,4-dimethyl-5,7-dihydropyrrolo[3,4-b]pyridin-6-yl)-[(3R)-1-(4-methoxypyrimidin-2-yl)pyrrolidin-3-yl]methanone;
(3-chloro-2,4-dimethyl-5,7-dihydropyrrolo[3,4-b]pyridin-6-yl)-[(3R)-1-(4-methylpyrimidin-2-yl)pyrrolidin-3-yl]methanone;
(3-chloro-2,4-dimethyl-5,7-dihydropyrrolo[3,4-b]pyridin-6-yl)-[(3R)-1-[4-(trifluoromethyl)pyrimidin-2-yl]pyrrolidin-3-yl]methanone;
(3-chloro-2,4-dimethyl-5,7-dihydropyrrolo[3,4-b]pyridin-6-yl)-[(3R)-1-(6-methylpyrazin-2-yl)pyrrolidin-3-yl]methanone;
(3-chloro-2,4-dimethyl-5,7-dihydropyrrolo[3,4-b]pyridin-6-yl)-[(3R)-1-(6-methylpyridazin-4-yl)pyrrolidin-3-yl]methanone;
[3-chloro-2-(methoxymethyl)-4-methyl-5,7-dihydropyrrolo[3,4-b]pyridin-6-yl]-[(3R)-1-(6-methylpyrimidin-4-yl)pyrrolidin-3-yl]methanone;
(3-chloro-2,4-dimethyl-5,7-dihydropyrrolo[3,4-b]pyridin-6-yl)-[(3R)-1-[6-(difluoromethyl)pyrimidin-4-yl]pyrrolidin-3-yl]methanone;
[3-chloro-2-(methoxymethyl)-4-methyl-5,7-dihydropyrrolo[3,4-b]pyridin-6-yl]-[(3R)-1-pyrimidin-4-ylpyrrolidin-3-yl]methanone;
[3-chloro-2-(methoxymethyl)-4-methyl-5,7-dihydropyrrolo[3,4-b]pyridin-6-yl]-[(3R)-1-(3-pyridyl)pyrrolidin-3-yl] methanone;
[3-chloro-2-(methoxymethyl)-4-methyl-5,7-dihydropyrrolo[3,4-b]pyridin-6-yl]-[(3R)-1-[2-(difluoromethyl)-4-pyridyl] pyrrolidin-3-yl]methanone;
[3-chloro-2-(methoxymethyl)-4-methyl-5,7-dihydropyrrolo[3,4-b]pyridin-6-yl]-[(3R)-1-pyrimidin-5-ylpyrrolidin-3-yl] methanone; and
[3-chloro-2-(methoxymethyl)-4-methyl-5,7-dihydropyrrolo[3,4-b]pyridin-6-yl]-[(3R)-1-pyridazin-4-ylpyrrolidin-3-yl] methanone.

In a preferred embodiment, there is provided a compound of formula (I) as described herein, or a pharmaceutically acceptable salt thereof, selected from:

(3-chloro-2,4-dimethyl-5,7-dihydropyrrolo[3,4-b]pyridin-6-yl)-[(3R)-1-(3-pyridyl)pyrrolidin-3-yl]methanone;
(3-chloro-2,4-dimethyl-5,7-dihydropyrrolo[3,4-b]pyridin-6-yl)-[(3R)-1-pyrimidin-4-ylpyrrolidin-3-yl]methanone;
(3-chloro-2,4-dimethyl-5,7-dihydropyrrolo[3,4-b]pyridin-6-yl)-[(3R)-1-[5-(difluoromethoxy)-3-pyridyl]pyrrolidin-3-yl] methanone;
(3-chloro-2,4-dimethyl-5,7-dihydropyrrolo[3,4-b]pyridin-6-yl)-[(3R)-1-[2-(difluoromethyl)-4-pyridyl]pyrrolidin-3-yl] methanone;
(3-chloro-2,4-dimethyl-5,7-dihydropyrrolo[3,4-b]pyridin-6-yl)-[(3R)-1-[2-(trifluoromethyl)-4-pyridyl]pyrrolidin-3-yl] methanone;
4-[(3R)-3-(3-chloro-2,4-dimethyl-5,7-dihydropyrrolo[3,4-b]pyridine-6-carbonyl)pyrrolidin-1-yl]pyrimidine-2-carbonitrile;
(3-chloro-2,4-dimethyl-5,7-dihydropyrrolo[3,4-b]pyridin-6-yl)-[(3R)-1-pyrimidin-5-ylpyrrolidin-3-yl]methanone;
(3-chloro-2,4-dimethyl-5,7-dihydropyrrolo[3,4-b]pyridin-6-yl)-[(3R)-1-[6-(difluoromethyl)pyrimidin-4-yl]pyrrolidin-3-yl]methanone;
3-chloro-2-(methoxymethyl)-4-methyl-5,7-dihydropyrrolo[3,4-b]pyridin-6-yl]-[(3R)-1-pyrimidin-4-ylpyrrolidin-3-yl] methanone; and
[3-chloro-2-(methoxymethyl)-4-methyl-5,7-dihydropyrrolo[3,4-b]pyridin-6-yl]-[(3R)-1-(3-pyridyl)pyrrolidin-3-yl] methanone.

Processes of manufacturing

Processes for the manufacture of compounds of formula (I), or pharmaceutically acceptable salts thereof, as described herein are also an object of the present invention.

The preparation of compounds of formula (I) as described herein may be carried out in sequential or convergent synthetic routes. Syntheses of the invention are shown in the following general schemes. The skills required for carrying out the reactions and purifications of the resulting products are known to those skilled in the art. The substituents and indices used in the following description of the processes have the significance given herein before unless indicated to the contrary.

If one of the starting materials, intermediates or compounds of formula (I) contain one or more functional groups which are not stable or are reactive under the reaction conditions of one or more reaction steps, appropriate protecting groups (as described e.g. in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), 9-fluorenylmethyl carbamate (Fmoc), 2-trimethylsilylethyl carbamate (Teoc), carbobenzyloxy (Cbz) and p-methoxybenzyloxycarbonyl (Moz).

If starting materials or intermediates contain stereogenic centers, compounds of formula (I) can be obtained as mixtures of diastereomers or enantiomers, which can be separated by methods well known in the art e.g., chiral HPLC, chiral SFC or chiral crystallization. Racemic compounds can, for example, be separated into their antipodes via diastereomeric salts by crystallization with optically pure acids or by separation of the antipodes by specific chromatographic methods using either a chiral adsorbent or a chiral eluent. It is equally possible to separate starting materials and intermediates containing stereogenic centers to afford diastereomerically/enantiomerically enriched starting materials and intermediates. Using such diastereomerically/enantiomerically enriched starting materials and intermediates in the synthesis of compounds of formula (I) will typically lead to the respective diastereomerically/enantiomerically enriched compounds of formula (I).

A person skilled in the art will acknowledge that the sequence of reactions may be varied depending on reactivity and nature of the intermediates.

In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 2nd Edition, Richard C. Larock. John Wiley & Sons, New York, NY. 1999*).* It was found convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux. The time required for the reaction may also vary widely, depending on many factors, notably the reaction temperature and the nature of the reagents. However, a period of from 0.5 hours to several days will usually suffice to yield the described intermediates and compounds. The reaction sequence is not limited to the one displayed in the schemes, however, depending on the starting materials and their respective reactivity, the sequence of reaction steps can be freely altered.

If starting materials or intermediates are not commercially available or their synthesis not described in literature, they can be prepared in analogy to existing procedures for close analogues or as outlined in the experimental section.

In one embodiment, compounds of formula (I), as described herein or a pharmaceutically acceptable salt thereof, may be prepared by a process comprising reacting an amine salt 1

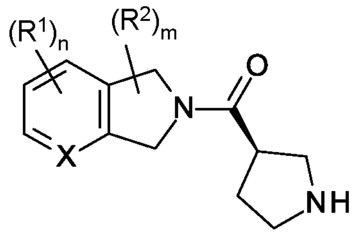

. 2HCl **1**

with a halide compound 2

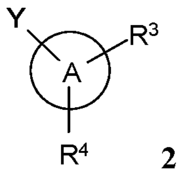

**2**

wherein $R^1$, $R^2$, $R^3$, $R^4$, X and A are as described herein, and Y is a halogen, to form said compound of formula (I), and optionally converting the compounds obtained into a pharmaceutically acceptable salt thereof.

In one embodiment, compounds of formula (I) wherein $R^1$, $R^2$, $R^3$ and $R^4$ are as described herein and their intermediates may be prepared in analogy to literature procedures and/or depicted for example in scheme 1 respectively.

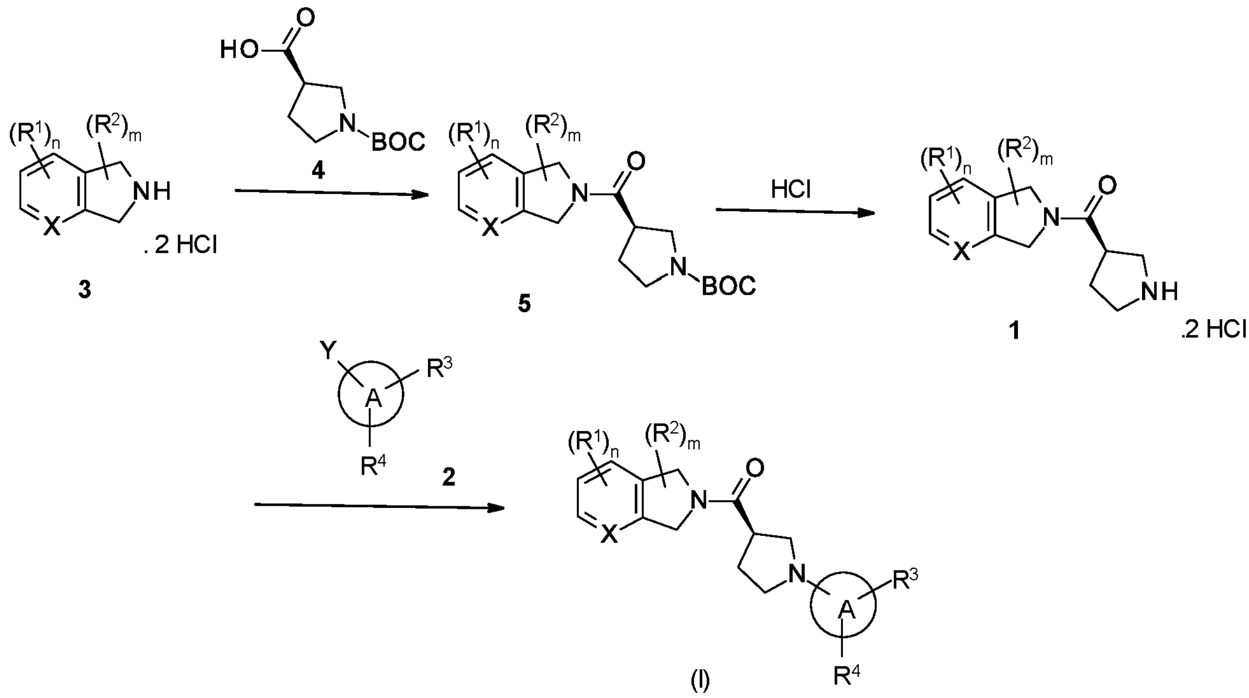


Scheme 1

The preparation of compounds of formula (I) may start with an amide coupling reaction between an amine derivative **3** and (R)-1-(tert-butoxycarbonyl)pyrrolidine-3-carboxylic acid **4** in the presence of a dehydrating agent such as TBTU and a base such as $i$Pr$_2$NEt to afford derivatives **5.** The preparation of amine derivatives **3** are either reported in the literature or described hereinafter. Upon deprotection with HCl the free amine intermediates **1** are obtained. Then a Buchwald cross coupling reaction (in the presence of a catalyst such as $Pd_2(dba)_3$ and a ligand such as tBuXPhos) or a thermal aromatic nucleophilic substitution reaction (in presence of a base such as $i$Pr$_2$NEt), between compound **1** and halide **2** (either commercially available or known in the literature so that they can be prepared by methods known in the art) is carried out to afford compounds of formula (I).

In one aspect, the present invention provides a compound of formula (I) as described herein, or a pharmaceutically acceptable salt thereof, when manufactured according to any one of the processes described herein.

Pharmaceutical compositions and administration

Another object of the present invention is a pharmaceutical composition comprising a compound of formula (I) as described herein, or a pharmaceutically acceptable salt thereof, and a pharmaceutically acceptable excipient.

The compounds of formula (I) and their pharmaceutically acceptable salts can be used as medicaments, in the form of pharmaceutical preparations. The pharmaceutical preparations can be administered internally, such as orally (e.g. in the form of tablets, coated tablets, dragées, hard and soft gelatine capsules, solutions, emulsions or suspensions), nasally (e.g. in the form of nasal sprays) or rectally (e.g. in the form of suppositories). However, the administration can also be effected parenterally, such as intramuscularly or intravenously (e.g. in the form of injection solutions). The administration can also be effected topically, e.g. transdermal administration, or in form of eye drops or ear drops.

The compounds of formula (I) and their pharmaceutically acceptable salts can be processed with pharmaceutically inert, inorganic or organic carriers for the production of pharmaceutical preparations, such as tablets, coated tablets, dragées, hard gelatin capsules, injection solutions or topical formulations. Lactose, corn starch or derivatives thereof, talc, stearic acids or salts thereof, and the like can be used, for example, as such carriers for tablets, coated tablets, dragées and hard gelatin capsules.

Suitable carriers for soft gelatin capsules are, for example, vegetable oils, waxes, fats, semi-solid substances and liquid polyols and the like. Depending on the nature of the active substance no carriers are, however, usually required in the case of soft gelatin capsules.

Suitable carriers for the production of solutions and syrups are, for example, water, alcohols, polyols, saccharose, glucose, invert sugar, vegetable oil, etc.

Suitable carriers for injection solutions are, for example, water, alcohols, polyols, glycerol, vegetable oils, etc.

Suitable carriers for suppositories are, for example, natural or hardened oils, waxes, fats, semi-liquid or liquid polyols, etc.

Suitable carriers for topical ocular formulations are, for example, cyclodextrins, mannitol or many other carriers and excipients known in the art.

Moreover, the pharmaceutical preparations can contain preservatives, solubilizers, viscosity increasing substances, stabilizers, wetting agents, emulsifiers, sweeteners, colorants, flavorants, salts for varying the osmotic pressure, buffers, masking agents or antioxidants. They can also contain other therapeutically valuable substances.

Medicaments containing a compound of formula (I) or a pharmaceutically acceptable salt thereof and a pharmaceutically acceptable excipient are also an object of the present invention, as is a process for their production, which comprises bringing one or more compounds of formula (I) and/or pharmaceutically acceptable salts thereof and, if desired, one or more other therapeutically valuable substances into a galenical administration form together with one or more pharmaceutically acceptable excipients.

The dosage can vary within wide limits and will, of course, have to be adjusted to the individual requirements in each particular case. In general, in the case of oral administration a daily dosage of about 0.1 mg to 20 mg per kg body weight, preferably 0.5 mg to 4 mg per kg body weight (e.g. about 300 mg per person), divided into preferably 1-3 individual doses, which can consist, for example, of the same amounts, should be appropriate. In the case of topical administration, the formulation can contain 0.001% to 15% by weight of medicament and the required dose, which can be between 0.1 and 25 mg, and can be administered either by single dose per day or per week, or by multiple doses (2 to 4) per day, or by multiple doses per week. It will, however, be clear that the upper or lower limit given herein can be exceeded when this is shown to be indicated.

The pharmaceutical composition according to the invention may be prepared as follows.

**Preparation of pharmaceutical compositions comprising compounds of the invention**

Tablet Formulation (Wet Granulation)

| **Ingredient** | **mg/tablet** | | | |
|---|---|---|---|---|
| | 5 | 25 | 100 | 500 |
| 1) Compound of formula (I) | 5 | 25 | 100 | 500 |
| 2) Lactose Anhydrous DTG | 125 | 105 | 30 | 150 |
| 3) Sta-Rx 1500 | 6 | 6 | 6 | 30 |
| 4) Microcrystalline Cellulose | 30 | 30 | 30 | 150 |
| 5) Magnesium Stearate | 1 | 1 | 1 | 1 |
| Total | 167 | 167 | 167 | 831 |

*Manufacturing Procedure:*

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

Capsule Formulation

| **Ingredient** | **mg/capsule** | | | |
|---|---|---|---|---|
| | 5 | 25 | 100 | 500 |
| 1) Compound of formula (I) | 5 | 25 | 100 | 500 |

(continued)

| Ingredient | mg/capsule | | | |
|---|---|---|---|---|
| | 5 | 25 | 100 | 500 |
| 2) Hydrous Lactose | 159 | 123 | 148 | - |
| 3) Corn Starch | 25 | 35 | 40 | 70 |
| 4) Talc | 10 | 15 | 10 | 25 |
| 5) Magnesium Stearate | 1 | 2 | 2 | 5 |
| Total | 200 | 200 | 300 | 600 |

*Manufacturing Procedure:*

1. Mix ingredients 1, 2 and 3 in a suitable mixer for 30 minutes.
2. Add ingredients 4 and 5 and mix for 3 minutes.
3. Fill into a suitable capsule.

Injection Solutions

| Ingredient | mg/injection solution |
|---|---|
| Compound of formula I | 3 |
| Polyethylene Glycol 400 | 150 |
| acetic acid | q.s. ad pH 5.0 |
| water for injection solutions | ad 1.0 ml |

*Manufacturing Procedure:*

A compound of formula (I) is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

Indications

Also an object of the present invention is a compound of formula (I) as described herein, or a pharmaceutically acceptable salt thereof, for use as therapeutically active substance.

As described above, compounds of formula (I) and their pharmaceutically acceptable salts are useful as M4 positive allosteric modulators.

In one aspect, the present invention provides compounds of formula (I) as described herein, or a pharmaceutically acceptable salt thereof, for use in the therapeutic and/or prophylactic treatment of Alzheimer's disease, schizophrenia, psychosis, Parkinson's disease, pain, addiction and Huntington's disease.

In a further aspect, the present disclosure provides the use of compounds of formula (I) as described herein, or a pharmaceutically acceptable salt thereof, for the therapeutic and/or prophylactic treatment of Alzheimer's disease, schizophrenia, psychosis, Parkinson's disease, pain, addiction and Huntington's disease.

In a further aspect, the present disclosure provides the use of compounds of formula (I) as described herein, or a pharmaceutically acceptable salt thereof, for the preparation of a medicament for the therapeutic and/or prophylactic treatment of Alzheimer's disease, schizophrenia, psychosis, Parkinson's disease, pain, addiction and Huntington's disease.

In a further aspect, the present disclosure provides a method for the therapeutic and/or prophylactic treatment of Alzheimer's disease, schizophrenia, psychosis, Parkinson's disease, pain, addiction and Huntington's disease, which method comprises administering an effective amount of a compound of formula (I) as described herein, or a pharmaceutically acceptable salt thereof.

**Examples**

*1) Preparative examples*

1.1) Preparation of intermediates

Intermediate 1-1:

**(3-chloro-2,4-dimethyl-5,7-dihydropyrrolo[3,4-b]pyridin-6-yl)-[(3R)-pyrrolidin-3-yl] methanone, dihydrochlorid salt.**

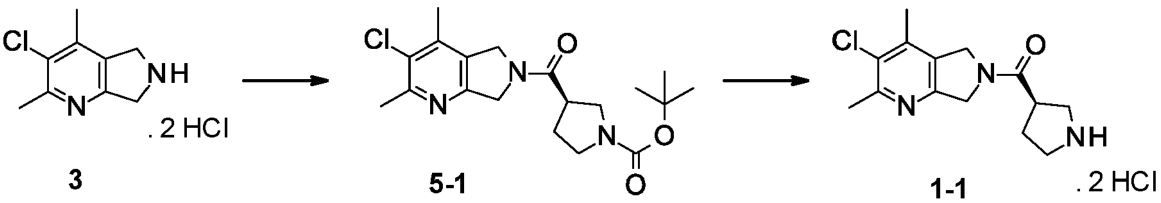

**3** **5-1** **1-1**

Step 1: To a colorless solution of (R)-1-(tert-butoxycarbonyl)pyrrolidine-3-carboxylic acid (2.53 g, 11.7 mmol, Eq: 1.2) in DMF (100 mL) were added TBTU (3.77 g, 11.7 mmol, Eq: 1.2) and *i*$Pr_2NEt$ (7.59 g, 10.3 mL, 58.7 mmol, Eq: 6). After five minutes, 3-chloro-2,4-dimethyl-6,7-dihydro-5H-pyrrolo[3,4-b]pyridine dihydrochloride (preparation described in literature in WO2018/002760, 2.5 g, 9.78 mmol, Eq: 1) was added and stirring was continued for an additional 6 hours. The mixture was concentrated under vacuum, and the residue dissolved in EtOAc (200 mL) and washed with an aqueous NaOH solution (1N, 80 mL). The organic phase was dried over $Na_2SO_4$ and concentrated under vacuum. The residue was purified with ISCO-chromatography ($SiO_2$, 80g, 0% to 10% 2N-$NH_3$-MeOH in $CH_2Cl_2$; Flow 60 ml/min; Collect by UV 254 nm; 280 nm) Cartridge type: RediSep® Rf Silica Gel (40-60 microns), to yield the intermediate **5-1**, compound tert-butyl (3R)-3-(3-chloro-2,4-dimethyl-5,7-dihydropyrrolo[3,4-b]pyridine-6-carbonyl)pyrrolidine-1-carboxylate as a white solid (3.05g, 8.02 mmol, 80% yield).

Step 2: To a white suspension of tert-butyl (3R)-3-(3-chloro-2,4-dimethyl-5,7-dihydropyrrolo[3,4-b]pyridine-6-carbonyl)pyrrolidine-1-carboxylate (3.05 g, 8.03 mmol, Eq. 1.0) in MeOH (55 mL) was added an HCl solution in dioxan (4M, 12 mL, 48 mmol, 6.0 Eq.) to give a colorless solution. Stirring was continued overnight at RT, and all volatiles were evaporated under vacuum to yield the title intermediate **1-1** as a white solid. (2.7 g, 97% yield). MS (ES+) *m*/*z:* 280.1 [$(M+H)^+$].

Intermediate **1-2:**

**[3-chloro-2-(methoxymethyl)-4-methyl-5,7-dihydropyrrolo[3,4-b]pyridin-6-yl]-[(3R)-pyrrolidin-3-yl]methanone, dihydrochlorid salt.**

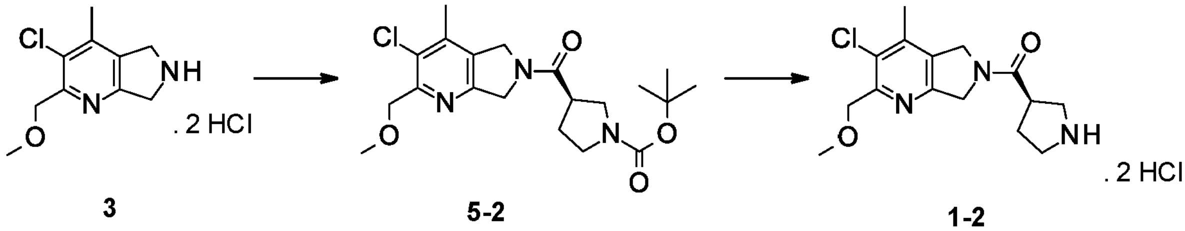

**3** **5-2** **1-2**

Step 1: To a colorless solution of (R)-1-(tert-butoxycarbonyl)pyrrolidine-3-carboxylic acid (208 mg, 0.97 mmol, Eq: 1.2) in DMF (10 mL) were added TBTU (310 mg, 0.97 mmol, Eq: 1.2) and *i*$Pr_2NEt$ (625 mg, 0.84 mL, 4.83 mmol, Eq: 6). After five minutes, 3-chloro-2-(methoxymethyl)-4-methyl-6,7-dihydro-5H-pyrrolo[3,4-b]pyridine dihydrochloride (preparation described in literature in WO2018/002760, 230 mg, 0.80 mmol, Eq: 1) was added and stirring was continued

for an additional 3 hours. The mixture was concentrated under vacuum, and the residue dissolved in EtOAc (10 mL) and washed with an aqueous NaOH solution (1N, 5 mL). The organic phase was dried over $Na_2SO_4$ and concentrated under vacuum. The residue was purified with ISCO-chromatography ($SiO_2$, 80g, 0% to 10% 2N-$NH_3$-MeOH in $CH_2Cl_2$; Flow 60 ml/min; Collect by UV 254 nm; 280 nm) Cartridge type: RediSep® Rf Silica Gel (40-60 microns), to yield the intermediate 5-2, compound tert-butyl (3R)-3-[3-chloro-2-(methoxymethyl)-4-methyl-5,7-dihydropyrrolo[3,4-b]pyridine-6-carbonyl] pyrrolidine-1-carboxylate as a light beige solid (300 mg, 90% yield).

Step 2: To a white suspension of tert-butyl (3R)-3-[3-chloro-2-(methoxymethyl)-4-methyl-5,7-dihydropyrrolo [3,4-b]pyridine-6-carbonyl]pyrrolidine-1-carboxylate (300 mg, 0.73 mmol, Eq. 1.0) in MeOH (5 mL) was added an HCl solution in dioxan (4M, 1.1 mL, 4.4 mmol, 6.0 Eq.) to give a colorless solution. Stirring was continued overnight at RT, and all volatiles were evaporated under vacuum to yield the title intermediate **1-2** as a white solid. (255 mg, 96% yield). MS (ES+) *m/z:* 310.2 [(M+H)+].

Intermediate **1-3:**

**[2-(methoxymethyl)-3,4-dimethyl-5,7-dihydropyrrolo[3,4-b]pyridin-6-yl]-[(3R)-pyrrolidin-3-yl]methanone, dihydrochlorid salt.**

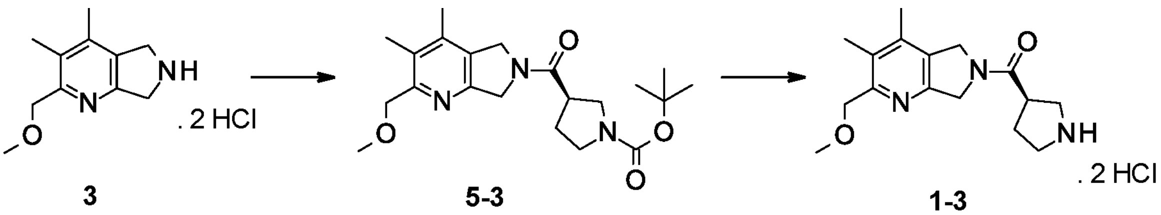

3 5-3 1-3

Step 1: To a colorless solution of (R)-1-(tert-butoxycarbonyl)pyrrolidine-3-carboxylic acid (68 mg, 0.32 mmol, Eq: 1.2) in DMF (5 mL) were added TBTU (102 mg, 0.32 mmol, Eq: 1.2) and *i*$Pr_2NEt$ (205 mg, 0.28 mL, 1.58 mmol, Eq: 6). After five minutes, 2-(methoxymethyl)-3,4-dimethyl-6,7-dihydro-5H-pyrrolo[3,4-b]pyridine dihydrochloride (preparation described in literature in WO 2018/002760, 70 mg, 0.26 mmol, Eq: 1) was added and stirring was continued for an additional 16 hours. The mixture was concentrated under vacuum, and the residue dissolved in EtOAc (5 mL) and washed with an aqueous NaOH solution (1N, 3 mL). The organic phase was dried over $Na_2SO_4$ and concentrated under vacuum. The residue was purified with ISCO-chromatography ($SiO_2$, 80g, 0% to 10% 2N-$NH_3$-MeOH in $CH_2Cl_2$; Flow 60 ml/min; Collect by UV 254 nm; 280 nm) Cartridge type: RediSep® Rf Silica Gel (40-60 microns), to yield the intermediate **5-3,** compound tert-butyl (3R)-3-[2-(methoxymethyl)-3,4-dimethyl-5,7-dihydropyrrolo[3,4-b]pyridine-6-carbonyl] pyrrolidine-1-carboxylate as a light beige solid (95 mg, 92% yield).

Step 2: To a white suspension of tert-butyl (3R)-3-[2-(methoxymethyl)-3,4-dimethyl-5,7-dihydropyrrolo[3,4-b] pyridine-6-carbonyl]pyrrolidine-1-carboxylate (95 mg, 0.24 mmol, Eq. 1.0) in MeOH (2 mL) was added an HCl solution in dioxan (4M, 0.37 mL, 1.4 mmol, 6.0 Eq.) to give a solution. Stirring was continued overnight at RT, and all volatiles were evaporated under vacuum to yield the title intermediate **1-3** as a light yellow solid. (79 mg, 100% yield). MS (ES+) *m/z:* 290.3 [(M+H)+].

Intermediate **1-4:**

**[2-(difluoromethyl)-3,4-dimethyl-5,7-dihydropyrrolo[3,4-b]pyridin-6-yl]-[(3R)-pyrrolidin-3-yl]methanone, dihydrochlorid salt.**

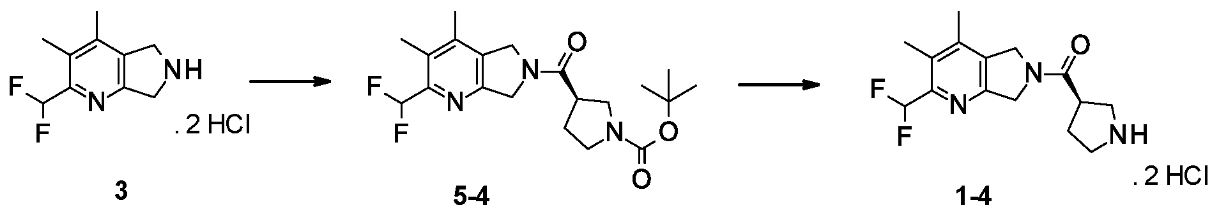

3 5-4 1-4

Step 1: To a colorless solution of (R)-1-(tert-butoxycarbonyl)pyrrolidine-3-carboxylic acid (312 mg, 1.45 mmol,

Eq: 1.2) in DMF (22 mL) were added TBTU (466 mg, 1.45 mmol, Eq: 1.2) and *i*$Pr_2NEt$ (938 mg, 1.27 mL, 7.26 mmol, Eq: 6). After five minutes, 2-(difluoromethyl)-3,4-dimethyl-6,7-dihydro-5H-pyrrolo[3,4-b]pyridine dihydrochloride (preparation described in literature in WO 2018/002760, 328 mg, 1.21 mmol, Eq: 1) was added and stirring was continued for additional 2 hours. The mixture was concentrated under vacuum, and the residue dissolved in EtOAc (10 mL) and washed with an aqueous NaOH solution (1N, 2 mL). The organic phase was dried over $Na_2SO_4$ and concentrated under vacuum. The residue was purified with ISCO-chromatography ($SiO_2$, 80g, 0% to 10% 2N-$NH_3$-MeOH in $CH_2Cl_2$; Flow 60ml/min; Collect by UV 254nm; 280nm) Cartridge type: RediSep® Rf Silica Gel (40-60 microns), to yield the intermediate **5-4,** compound tert-butyl (3R)-3-[2-(difluoromethyl)-3,4-dimethyl-5,7-dihydropyrrolo[3,4-b]pyridine-6-carbonyl] pyrrolidine-1-carboxylate as an off-white solid (490 mg, 98% yield).

Step 2: To a white suspension of tert-butyl (3R)-3-[2-(difluoromethyl)-3,4-dimethyl-5,7-dihydropyrrolo[3,4-b] pyridine-6-carbonyl]pyrrolidine-1-carboxylate (490 mg, 1.24 mmol, Eq. 1.0) in MeOH (10 mL) was added an HCl solution in dioxan (4M, 1.86 mL, 7.4 mmol, 6.0 Eq.) to give a solution. Stirring was continued overnight at RT, and all volatiles were evaporated under vacuum to yield the title intermediate **1-4** as a light yellow solid. (450 mg, 100% yield). MS (ES+) *m/z:* 296.2 [$(M+H)^+$].

1.2) General procedures

1.2.1) Buchwald coupling reaction

In a sealed tube, to a suspension of an intermediate **1** (1 mmol) in 2-Me-THF (10 mL) were added 1.1 equivalent of halide **2** and NaO*t*Bu (3.0 eq.) at RT, degassed and stirred for 5 minutes under argon before *t*Bu-Xphos (0.1 eq.) and $Pd_2(dba)_3$ (0.05 eq.) were added. The reaction mixture was heated to 60°C for 45 minutes, cooled down to RT, filtered through a short plug of $SiO_2$-$NH_2$, washed with EtOAc/MeOH 9:1 (6 x 10 ml) and concentrated in vacuo. A purification was done either by flash column chromatography or reverse phase preparative HPLC to afford the desired compound of formula (I).

1.2.2) Nucleophilic aromatic substitution

In a sealed tube, to a suspension of an intermediate **1** (1 mmol) in DMA (10 mL) was added *i*$Pr_2Net$ (5.0 eq.). To this solution under argon, was added halide **2** (2.0 eq.) and the reaction mixture heated to 95°C for 30 minutes. The reaction mixture was cooled down to RT, concentrated under vacuum and a purification was done either by flash column chromatography or reverse phase preparative HPLC to afford the desired compound of formula (I).

**Example 1**

**(3-chloro-2,4-dimethyl-5,7-dihydropyrrolo[3,4-b]pyridin-6-yl)-[(3R)-1-(3-pyridyl)pyrrolidin-3-yl]methanone**

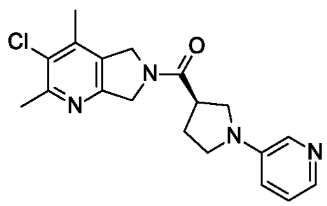

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromopyridine, afforded the title product (55% yield) as white solid, MS (ES+) *m/z:* 357.1 [$(M+H)^+$].

**Example 2**

**(3-chloro-2,4-dimethyl-5,7-dihydropyrrolo[3,4-b]pyridin-6-yl)-[(3R)-1-pyrimidin-4-ylpyrrolidin-3-yl]methanone**

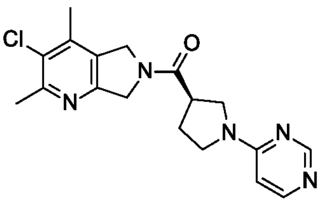

An aromatic nucleophilic substitution reaction using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-chloropyrimidine, afforded the title product (69% yield) as white solid, MS (ES+) *m/z:* 358.1 [$(M+H)^+$].

**Example 3**

**(3-chloro-2,4-dimethyl-5,7-dihydropyrrolo[3,4-b]pyridin-6-yl)-[(3R)-1-(4-pyridyl)pyrrolidin-3-yl]methanone**

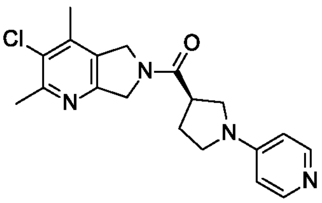

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromopyridine, afforded the title product (48% yield) as white solid, MS (ES+) *m/z:* 357.2 [$(M+H)^+$].

**Example 4**

**(3-chloro-2,4-dimethyl-5,7-dihydropyrrolo[3,4-b]pyridin-6-yl)-[(3R)-1-(6-methyl-3-pyridyl)pyrrolidin-3-yl]methanone**

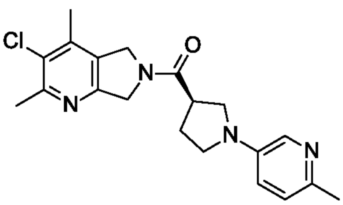

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-2-methyl-pyridine, afforded the title product (72% yield) as white solid, MS (ES+) *m/z:* 371.2 [$(M+H)^+$].

**Example 5**

**(3-chloro-2,4-dimethyl-5,7-dihydropyrrolo[3,4-b]pyridin-6-yl)-[(3R)-1-(2-methyl-4-pyridyl)pyrrolidin-3-yl]methanone**

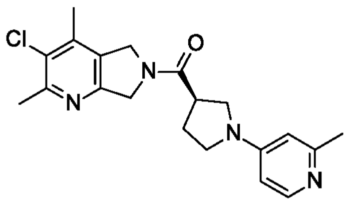

A Buchwald type coupling using the general procedure as described above, between (3-chloro-2,4-dimethyl-5,7-dihydropyrrolo[3,4-b]pyridin-6-yl)-[(3R)-pyrrolidin-3-yl]methanone, dihydrochlorid salt **1-1** and 4-bromo-2-methyl-pyridine, afforded the title product (49% yield) as white solid, MS (ES+) *m/z:* 371.2 [(M+H)+].

**Example 6**

**(3-chloro-2,4-dimethyl-5,7-dihydropyrrolo[3,4-b]pyridin-6-yl)-[(3R)-1-(2-cyclopropylpyrimidin-4-yl)pyrrolidin-3-yl]methanone**

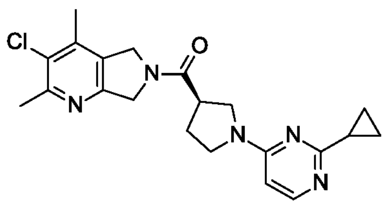

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromo-2-cyclopropyl-pyrimidine, afforded the title product (33% yield) as white solid, MS (ES+) *m/z:* 398.2 [(M+H)+].

**Example 7**

**(3-chloro-2,4-dimethyl-5,7-dihydropyrrolo[3,4-b]pyridin-6-yl)-[(3R)-1-[5-(trifluoromethyl)pyrimidin-2-yl]pyrrolidin-3-yl]methanone**

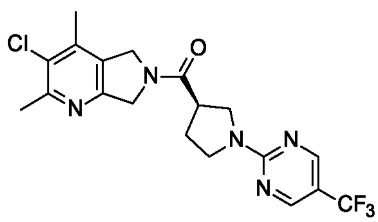

An aromatic nucleophilic substitution reaction using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 2-chloro-5-(trifluoromethyl)pyrimidine, afforded the title product (71% yield) as white solid, MS (ES+) *m/z:* 426.2 [(M+H)+].

**Example 8**

**(3-chloro-2,4-dimethyl-5,7-dihydropyrrolo[3,4-b]pyridin-6-yl)-[(3R)-1-(5-methylpyrimidin-2-yl)pyrrolidin-3-yl] methanone**

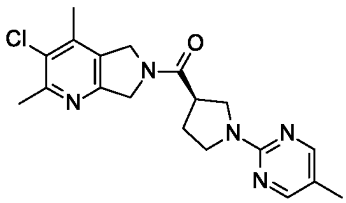

An aromatic nucleophilic substitution reaction using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 2-chloro-5-methyl-pyrimidine, afforded the title product (39% yield) as white solid, MS (ES+) *m/z:* 372.2 [(M+H)$^+$].

**Example 9**

**(3-chloro-2,4-dimethyl-5,7-dihydropyrrolo[3,4-b]pyridin-6-yl)-[(3R)-1-[6-(trifluoromethyl)-3-pyridyl]pyrrolidin-3-yl]methanone**

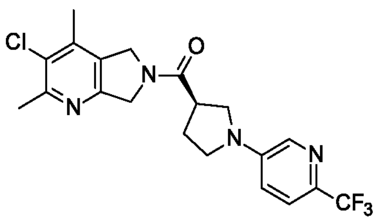

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-2-(trifluoromethyl)pyridine, afforded the title product (27% yield) as white solid, MS (ES+) *m/z:* 425.2 [(M+H)$^+$].

**Example 10**

**(3-chloro-2,4-dimethyl-5,7-dihydropyrrolo[3,4-b]pyridin-6-yl)-[(3R)-1-(6-methoxy-3-pyridyl)pyrrolidin-3-yl] methanone**

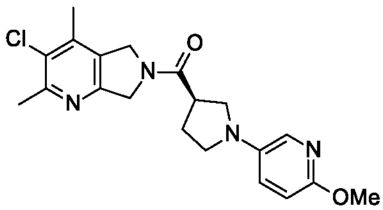

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-2-methoxy-pyridine, afforded the title product (42% yield) as white solid, MS (ES+) *m/z:* 387.1 [(M+H)$^+$].

**Example 11**

**(3-chloro-2,4-dimethyl-5,7-dihydropyrrolo[3,4-b]pyridin-6-yl)-[(3R)-1-[6-(methylamino)-3-pyridyl]pyrrolidin-3-yl]methanone**

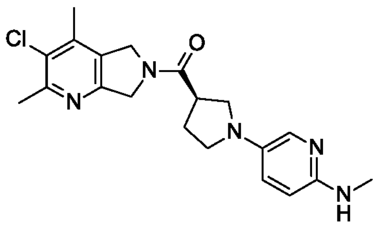

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-N-methyl-pyridin-2-amine, afforded the title product (20% yield) as white solid, MS (ES+) *m/z:* 386.2 [$(M+H)^+$].

**Example 12**

**(3-chloro-2,4-dimethyl-5,7-dihydropyrrolo[3,4-b]pyridin-6-yl)-[(3R)-1-(2-methyl-3-pyridyl)pyrrolidin-3-yl]methanone**

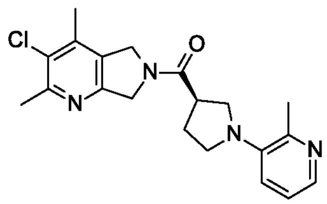

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt 1-1 and 3-bromo-2-methyl-pyridine, afforded the title product (34% yield) as white solid, MS (ES+) *m/z:* 371.1 [$(M+H)^+$].

**Example 13**

**(3-chloro-2,4-dimethyl-5,7-dihydropyrrolo[3,4-b]pyridin-6-yl)-[(3R)-1-(4-methyl-3-pyridyl)pyrrolidin-3-yl]methanone**

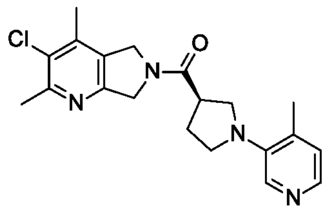

A Buchwald type coupling using the general procedure 1 as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromo-4-methyl-pyridine, afforded the title product (34% yield) as white solid, MS (ES+) *m/z:* 371.1 [$(M+H)^+$].

**Example 14**

**(3-chloro-2,4-dimethyl-5,7-dihydropyrrolo[3,4-b]pyridin-6-yl)-[(3R)-1-[5-(trifluoromethyl)-3-pyridyl]pyrrolidin-3-yl]methanone**

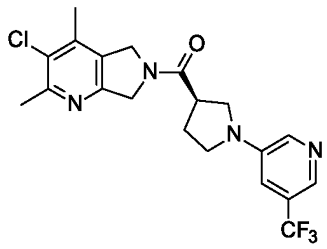

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromo-5-(trifluoromethyl)pyridine, afforded the title product (30% yield) as white solid, MS (ES+) *m/z:* 425.2 [$(M+H)^+$].

**Example 15**

**(3-chloro-2,4-dimethyl-5,7-dihydropyrrolo[3,4-b]pyridin-6-yl)-[(3R)-1-[5-(difluoromethyl)-3-pyridyl]pyrrolidin-3-yl]methanone**

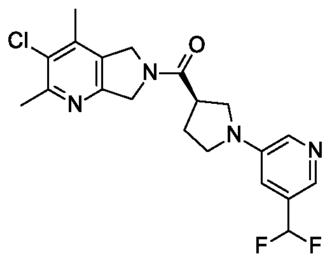

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt 1-1 and 3-bromo-5-(difluoromethyl) pyridine, afforded the title product (42% yield) as white solid, MS (ES+) *m/z:* 407.2 [$(M+H)^+$].

**Example 16**

**(3-chloro-2,4-dimethyl-5,7-dihydropyrrolo[3,4-b]pyridin-6-yl)-[(3R)-1-(5-cyclopropyl-3-pyridyl)pyrrolidin-3-yl] methanone**

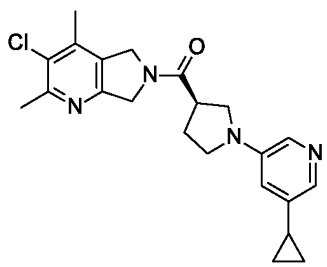

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromo-5-cyclopropylpyridine, afforded the title product (53% yield) as white solid, MS (ES+) *m/z:* 397.2 [$(M+H)^+$].

**Example 17**

**(3-chloro-2,4-dimethyl-5,7-dihydropyrrolo[3,4-b]pyridin-6-yl)-[(3R)-1-[5-(difluoromethoxy)-3-pyridyl]pyrrolidin-3-yl]methanone**

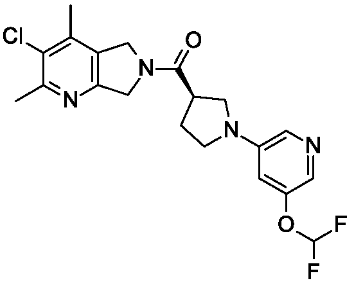

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromo-5-(difluoromethoxy)pyridine, afforded the title product (33% yield) as white solid, MS (ES+) *m*/*z:* 423.2 [(M+H)$^+$].

**Example 18**

**(3-chloro-2,4-dimethyl-5,7-dihydropyrrolo[3,4-b]pyridin-6-yl)-[(3R)-1-(5-methoxy-3-pyridyl)pyrrolidin-3-yl] methanone**

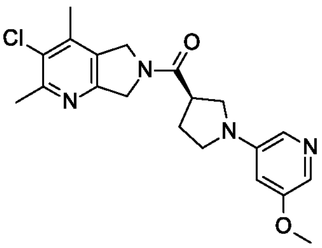

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromo-5-methoxy-pyridine, afforded the title product (46% yield) as white solid, MS (ES+) *m*/*z:* 387.1 [(M+H)$^+$].

**Example 19**

**(3-chloro-2,4-dimethyl-5,7-dihydropyrrolo[3,4-b]pyridin-6-yl)-[(3R)-1-(5-methylsulfonyl-3-pyridyl)pyrrolidin-3-yl]methanone**

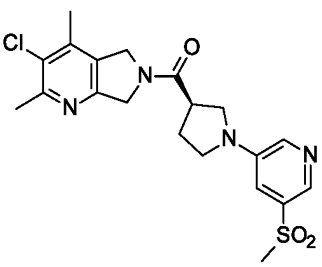

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromo-5-methylsulfonyl-pyridine, afforded the title product (31% yield) as white solid, MS (ES+) *m/z:* 435.1 [(M+H)+].

**Example 20**

**(3-chloro-2,4-dimethyl-5,7-dihydropyrrolo[3,4-b]pyridin-6-yl)-[(3R)-1-[2-(difluoromethyl)-4-pyridyl]pyrrolidin-3-yl]methanone**

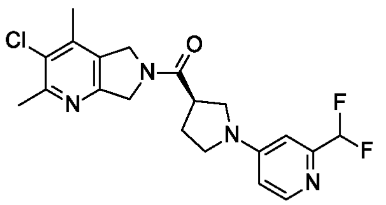

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromo-2-(difluoromethyl)pyridine, afforded the title product (54% yield) as white solid, MS (ES+) *m/z:* 407.1 [(M+H)+].

**Example 21**

**(3-chloro-2,4-dimethyl-5,7-dihydropyrrolo[3,4-b]pyridin-6-yl)-[(3R)-1-[2-(trifluoromethyl)-4-pyridyl]pyrrolidin-3-yl]methanone**

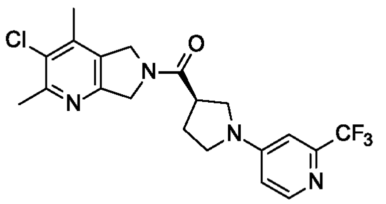

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromo-2-(trifluoromethyl)pyridine, afforded the title product (36% yield) as white solid, MS (ES+) *m/z:* 425.2 [(M+H)+].

**Example 22**

**(3-chloro-2,4-dimethyl-5,7-dihydropyrrolo[3,4-b]pyridin-6-yl)-[(3R)-1-(2-methoxy-4-pyridyl)pyrrolidin-3-yl] methanone**

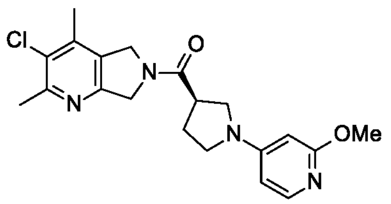

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromo-2-methoxy-pyridine, afforded the title product (51% yield) as white solid, MS (ES+) *m/z:* 387.1 [(M+H)+].

**Example 23**

**(3-chloro-2,4-dimethyl-5,7-dihydropyrrolo[3,4-b]pyridin-6-yl)-[(3R)-1-[2-(trifluoromethyl)pyrimidin-4-yl]pyrrolidin-3-yl]methanone**

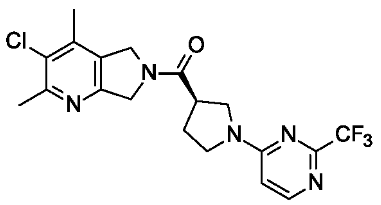

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromo-2-(trifluoromethyl)pyrimidine, afforded the title product (26% yield) as white solid, MS (ES+) *m/z:* 426.1 [$(M+H)^+$].

**Example 24**

**4-[(3R)-3-(3-chloro-2,4-dimethyl-5,7-dihydropyrrolo[3,4-b]pyridine-6-carbonyl)pyrrolidin-1-yl]pyrimidine-2-carbonitrile**

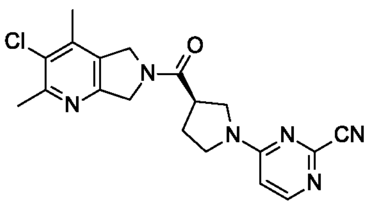

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromopyrimidine-2-carbonitrile, afforded the title product (14% yield) as white solid, MS (ES+) *m/z:* 383.2 [$(M+H)^+$].

**Example 25**

**(3-chloro-2,4-dimethyl-5,7-dihydropyrrolo[3,4-b]pyridin-6-yl)-[(3R)-1-(2-methoxypyrimidin-4-yl)pyrrolidin-3-yl] methanone**

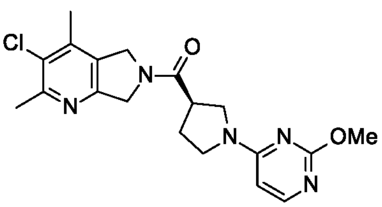

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromo-2-methoxy-pyrimidine, afforded the title product (10% yield) as white solid, MS (ES+) *m/z:* 388.2 [$(M+H)^+$].

**Example 26**

**(3-chloro-2,4-dimethyl-5,7-dihydropyrrolo[3,4-b]pyridin-6-yl)-[(3R)-1-(6-methylpyrimidin-4-yl)pyrrolidin-3-yl] methanone**

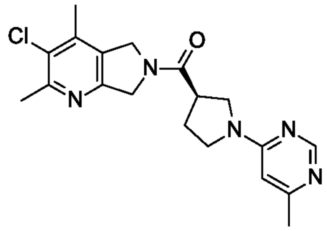

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromo-6-methyl-pyrimidine, afforded the title product (27% yield) as white solid, MS (ES+) *m/z:* 372.2 [$(M+H)^+$].

**Example 27**

**(3-chloro-2,4-dimethyl-5,7-dihydropyrrolo[3,4-b]pyridin-6-yl)-[(3R)-1-[6-(trifluoromethyl)pyrimidin-4-yl]pyrrolidin-3-yl]methanone**

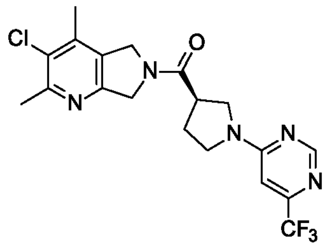

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromo-6-(trifluoromethyl)pyrimidine, afforded the title product (24% yield) as white solid, MS (ES+) *m/z:* 426.3 [$(M+H)^+$].

**Example 28**

**(3-chloro-2,4-dimethyl-5,7-dihydropyrrolo[3,4-b]pyridin-6-yl)-[(3R)-1-(6-cyclopropylpyrimidin-4-yl)pyrrolidin-3-yl]methanone**

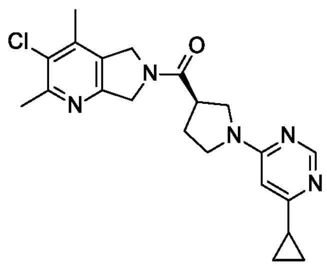

A Buchwald type coupling using the general procedure as described above, between (3-chloro-2,4-dimethyl-5,7-dihydropyrrolo[3,4-b]pyridin-6-yl)-[(3R)-pyrrolidin-3-yl]methanone, dihydrochlorid salt **1-1** and 4-bromo-6-cyclopropylpyrimidine, afforded the title product (38% yield) as white solid, MS (ES+) *m/z:* 398.2 [$(M+H)^+$].

**Example 29**

**(3-chloro-2,4-dimethyl-5,7-dihydropyrrolo[3,4-b]pyridin-6-yl)-[(3R)-1-(6-methoxypyrimidin-4-yl)pyrrolidin-3-yl] methanone**

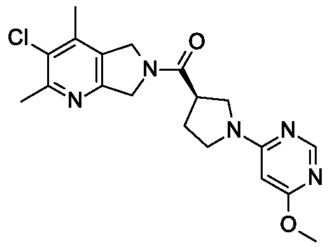

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromo-6-cyclopropyl-pyrimidine, afforded the title product (37% yield) as white solid, MS (ES+) *m/z:* 388.1 [(M+H)$^+$].

**Example 30**

**(3-chloro-2,4-dimethyl-5,7-dihydropyrrolo[3,4-b]pyridin-6-yl)-[(3R)-1-pyrimidin-5-ylpyrrolidin-3-yl]methanone**

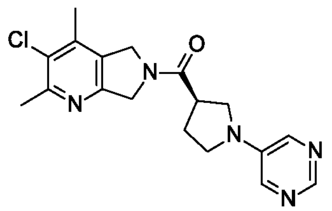

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromopyrimidine, afforded the title product (33% yield) as white solid, MS (ES+) *m/z:* 358.1 [(M+H)$^+$].

**Example 31**

**(3-chloro-2,4-dimethyl-5,7-dihydropyrrolo[3,4-b]pyridin-6-yl)-[(3R)-1-(2-methylpyrimidin-5-yl)pyrrolidin-3-yl] methanone**

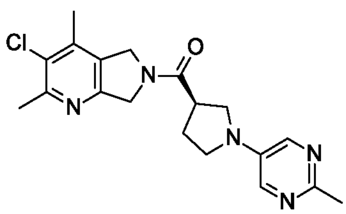

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-2-methyl-pyrimidine, afforded the title product (29% yield) as white solid, MS (ES+) *m/z:* 372.1 [(M+H)$^+$].

**Example 32**

**(3-chloro-2,4-dimethyl-5,7-dihydropyrrolo[3,4-b]pyridin-6-yl)-[(3R)-1-[2-(trifluoromethyl)pyrimidin-5-yl]pyrrolidin-3-yl]methanone**

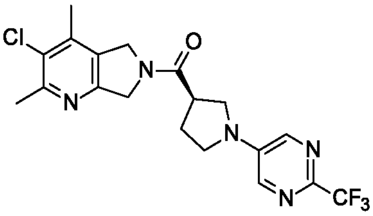

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-2-(trifluoromethyl)pyrimidine, afforded the title product (21% yield) as white solid, MS (ES+) *m/z:* 426.2 [(M+H)$^+$].

**Example 33**

**(3-chloro-2,4-dimethyl-5,7-dihydropyrrolo[3,4-b]pyridin-6-yl)-[(3R)-1-(2-methoxypyrimidin-5-yl)pyrrolidin-3-yl] methanone**

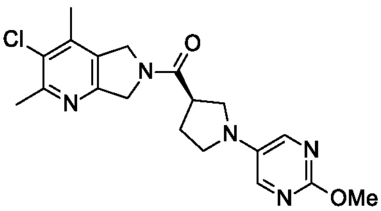

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-2-methoxy-pyrimidine, afforded the title product (46% yield) as white solid, MS (ES+) *m/z:* 388.1 [(M+H)$^+$].

**Example 34**

**(3-chloro-2,4-dimethyl-5,7-dihydropyrrolo[3,4-b]pyridin-6-yl)-[(3R)-1-[2-(oxetan-3-yl)pyrimidin-5-yl]pyrrolidin-3-yl]methanone**

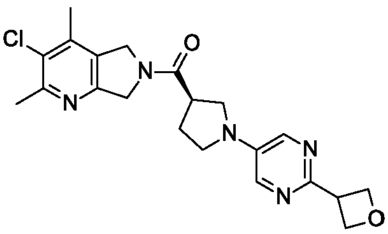

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-2-(oxetan-3-yl) pyrimidine, afforded the title product (47% yield) as white solid, MS (ES+) *m/z:* 414.1 [(M+H)$^+$].

**Example 35**

**(3-chloro-2,4-dimethyl-5,7-dihydropyrrolo[3,4-b]pyridin-6-yl)-[(3R)-1-[2-(methylamino)pyrimidin-5-yl]pyrrolidin-3-yl] methanone**

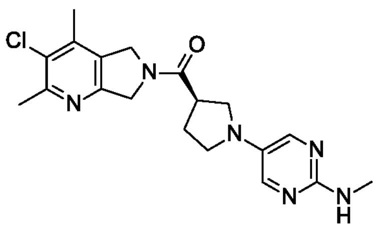

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-N-methyl-pyrimidin-2-amine, afforded the title product (18% yield) as white solid, MS (ES+) *m/z:* 387.2 [$(M+H)^+$].

**Example 36**

**(3-chloro-2,4-dimethyl-5,7-dihydropyrrolo[3,4-b]pyridin-6-yl)-[(3R)-1-[2-(dimethylamino)pyrimidin-5-yl]pyrrolidin-3-yl]methanone**

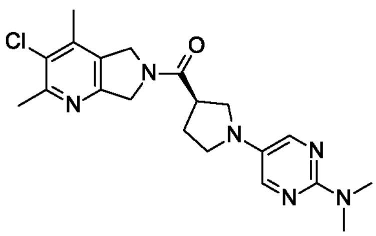

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-N,N-dimethyl-pyrimidin-2-amine, afforded the title product (43% yield) as white solid, MS (ES+) *m/z:* 401.2 [$(M+H)^+$].

**Example 37**

**(3-chloro-2,4-dimethyl-5,7-dihydropyrrolo[3,4-b]pyridin-6-yl)-[(3R)-1-(4-methylpyrimidin-5-yl)pyrrolidin-3-yl] methanone**

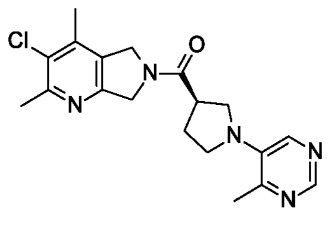

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromo-4-methyl-pyrimidine, afforded the title product (24% yield) as white solid, MS (ES+) *m/z:* 372.2 [$(M+H)^+$].

**Example 38**

**(3-chloro-2,4-dimethyl-5,7-dihydropyrrolo[3,4-b]pyridin-6-yl)-[(3R)-1-pyridazin-4-ylpyrrolidin-3-yl]methanone**

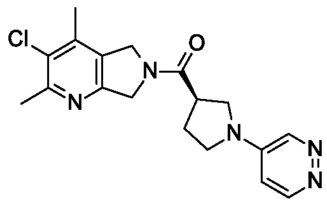

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 4-bromopyridazine, afforded the title product (34% yield) as white solid, MS (ES+) *m/z:* 358.2 [$(M+H)^+$].

**Example 39**

**(3-chloro-2,4-dimethyl-5,7-dihydropyrrolo[3,4-b]pyridin-6-yl)-[(3R)-1-pyridazin-3-ylpyrrolidin-3-yl]methanone**

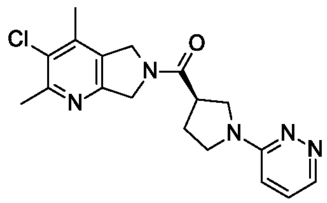

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromopyridazine, afforded the title product (16% yield) as white solid, MS (ES+) *m/z:* 358.2 [$(M+H)^+$].

**Example 40**

**(3-chloro-2,4-dimethyl-5,7-dihydropyrrolo[3,4-b]pyridin-6-yl)-[(3R)-1-(6-methylpyridazin-3-yl)pyrrolidin-3-yl] methanone**

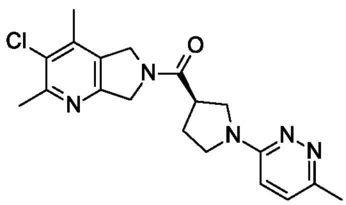

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromo-6-methyl-pyridazine, afforded the title product (30% yield) as white solid, MS (ES+) *m/z:* 372.1 [$(M+H)^+$].

**Example 41**

**(3-chloro-2,4-dimethyl-5,7-dihydropyrrolo[3,4-b]pyridin-6-yl)-[(3R)-1-[6-(trifluoromethyl)pyridazin-3-yl]pyrrolidin-3-yl]methanone**

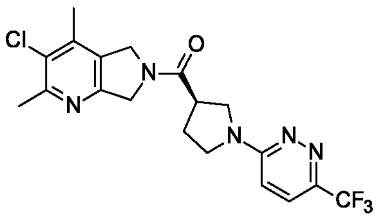

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 3-bromo-6-(trifluoromethyl)pyridazine, afforded the title product (21% yield) as white solid, MS (ES+) *m/z:* 426.2 [(M+H)$^+$].

**Example 42**

**(3-chloro-2,4-dimethyl-5,7-dihydropyrrolo[3,4-b]pyridin-6-yl)-[(3R)-1-(6-methoxypyrazin-2-yl)pyrrolidin-3-yl] methanone**

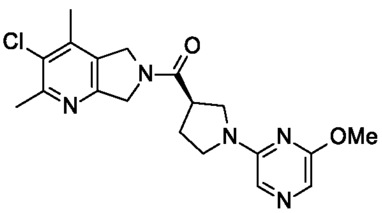

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 2-bromo-6-methoxy-pyrazine, afforded the title product (31% yield) as white solid, MS (ES+) *m/z:* 388.1 [(M+H)$^+$].

**Example 43**

**(3-chloro-2,4-dimethyl-5,7-dihydropyrrolo[3,4-b]pyridin-6-yl)-[(3R)-1-(6-cyclopropylpyrazin-2-yl)pyrrolidin-3-yl] methanone**

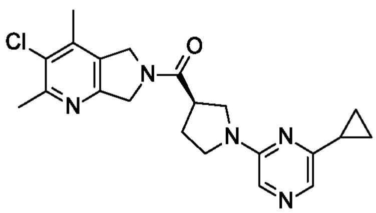

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 2-bromo-6-cyclopropylpyrazine, afforded the title product (10% yield) as white solid, MS (ES+) *m/z:* 398.3 [(M+H)$^+$].

**Example 44**

**5-[(3R)-3-(3-chloro-2,4-dimethyl-5,7-dihydropyrrolo[3,4-b]pyridine-6-carbonyl)pyrrolidin-1-yl]pyrazine-2-carbonitrile**

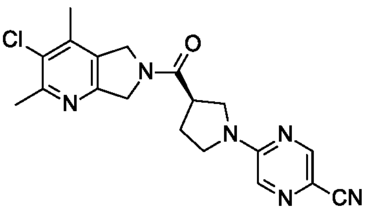

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 5-bromopyrazine-2-carbonitrile, afforded the title product (6% yield) as white solid, MS (ES+) *m/z:* 383.2 [(M+H)$^+$].

**Example 45**

**(3-chloro-2,4-dimethyl-5,7-dihydropyrrolo[3,4-b]pyridin-6-yl)-[(3R)-1-(5-methoxypyrazin-2-yl)pyrrolidin-3-yl] methanone**

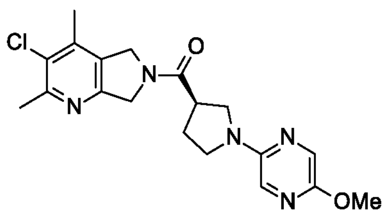

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 2-bromo-5-methoxy-pyrazine, afforded the title product (18% yield) as white solid, MS (ES+) *m/z:* 388.2 [(M+H)$^+$].

**Example 46**

**(3-chloro-2,4-dimethyl-5,7-dihydropyrrolo[3,4-b]pyridin-6-yl)-[(3R)-1-(5-methylpyrazin-2-yl)pyrrolidin-3-yl] methanone**

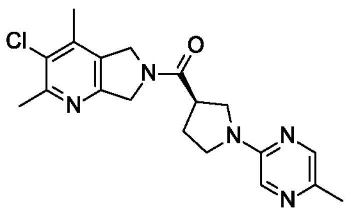

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 2-bromo-5-methyl-pyrazine, afforded the title product (10% yield) as white solid, MS (ES+) *m/z:* 372.2 [(M+H)$^+$].

**Example 47**

**(3-chloro-2,4-dimethyl-5,7-dihydropyrrolo[3,4-b]pyridin-6-yl)-[(3R)-1-[5-(trifluoromethyl)pyrazin-2-yl] pyrrolidin-3-yl] methanone**

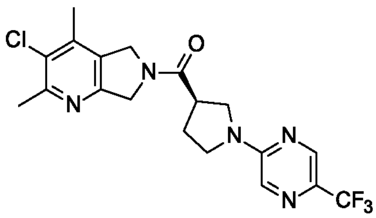

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt **1-1** and 2-bromo-5-(trifluoromethyl)pyrazine, afforded the title product (22% yield) as white solid, MS (ES+) *m/z:* 426.1 [$(M+H)^+$].

**Example 48**

**(3-chloro-2,4-dimethyl-5,7-dihydropyrrolo[3,4-b]pyridin-6-yl)-[(3R)-1-(4-methoxypyrimidin-2-yl)pyrrolidin-3-yl] methanone**

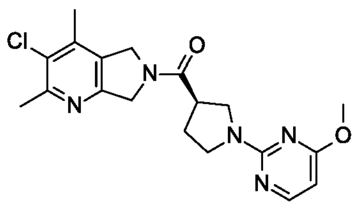

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt 1-1 and 2-bromo-4-methoxy-pyrimidine, afforded the title product (22% yield) as white solid, MS (ES+) *m/z:* 388.1 [$(M+H)^+$].

**Example 49**

**(3-chloro-2,4-dimethyl-5,7-dihydropyrrolo[3,4-b]pyridin-6-yl)-[(3R)-1-(4-methylpyrimidin-2-yl)pyrrolidin-3-yl] methanone**

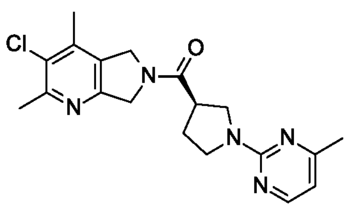

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt 1-1 and 2-bromo-4-methyl-pyrimidine, afforded the title product (34% yield) as white solid, MS (ES+) *m/z:* 372.1 [$(M+H)^+$].

**Example 50**

**(3-chloro-2,4-dimethyl-5,7-dihydropyrrolo[3,4-b]pyridin-6-yl)-[(3R)-1-[4-(trifluoromethyl)pyrimidin-2-yl] pyrrolidin-3-yl] methanone**

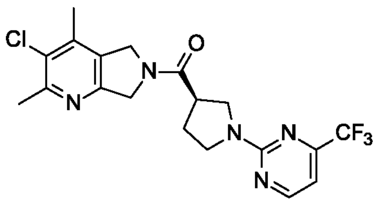

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt 1-1 and 2-bromo-4-(trifluoromethyl)pyrimidine, afforded the title product (9% yield) as white solid, MS (ES+) *m/z:* 426.2 [(M+H)$^+$].

**Example 51**

**(3-chloro-2,4-dimethyl-5,7-dihydropyrrolo[3,4-b]pyridin-6-yl)-[(3R)-1-(6-methylpyrazin-2-yl)pyrrolidin-3-yl] methanone**

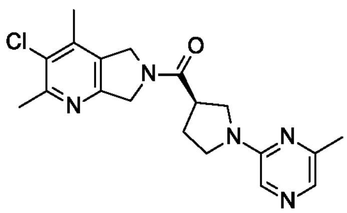

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt 1-1 and 2-bromo-6-methyl-pyrazine, afforded the title product (21% yield) as white solid, MS (ES+) *m/z:* 372.1 [(M+H)$^+$].

**Example 52**

**(3-chloro-2,4-dimethyl-5,7-dihydropyrrolo[3,4-b]pyridin-6-yl)-[(3R)-1-(6-methylpyridazin-4-yl)pyrrolidin-3-yl] methanone**

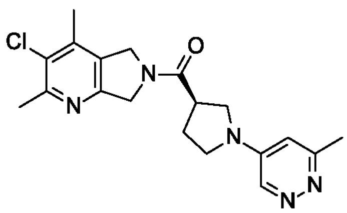

A Buchwald type coupling using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt 1-1 and 5-bromo-3-methyl-pyridazine, afforded the title product (48% yield) as white solid, MS (ES+) *m/z:* 372.1 [(M+H)$^+$].

**Example 53**

**[3-chloro-2-(methoxymethyl)-4-methyl-5,7-dihydropyrrolo[3,4-b]pyridin-6-yl]-[(3R)-1-(6-methylpyrimidin-4-yl) pyrrolidin-3-yl]methanone**

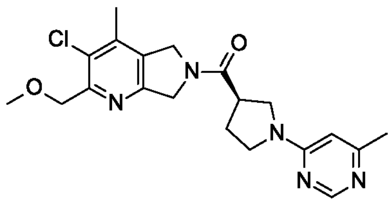

A Buchwald type coupling using the general procedure as described above, between [3-chloro-2-(methoxymethyl)-4-methyl-5,7-dihydropyrrolo[3,4-b]pyridin-6-yl]-[(3R)-pyrrolidin-3-yl]methanone, dihydrochlorid salt 1-2 and 4-bromo-6-methyl-pyrimidine, afforded the title product (34% yield) as white solid, MS (ES+) *m/z:* 402.2 [(M+H)$^+$].

**Example 54**

**(3-chloro-2,4-dimethyl-5,7-dihydropyrrolo[3,4-b]pyridin-6-yl)-[(3R)-1-[6-(difluoromethyl)pyrimidin-4-yl]pyrrolidin-3-yl]methanone**

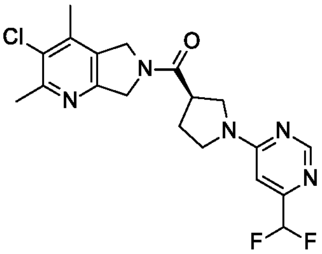

An aromatic nucleophilic substitution reaction using the general procedure as described above, between (3-chloro-2,4-dimethyl-5,7-dihydropyrrolo[3,4-b]pyridin-6-yl)-[(3R)-pyrrolidin-3-yl] methanone, dihydrochlorid salt 1-1 and 4-bromo-6-(difluoromethyl)pyrimidine, afforded the title product (82% yield) as white solid, MS (ES+) *m/z:* 408.1 [(M+H)$^+$].

**Example 55**

**[3-chloro-2-(methoxymethyl)-4-methyl-5,7-dihydropyrrolo[3,4-b]pyridin-6-yl]-[(3R)-1-pyrimidin-4-ylpyrrolidin-3-yl]methanone**

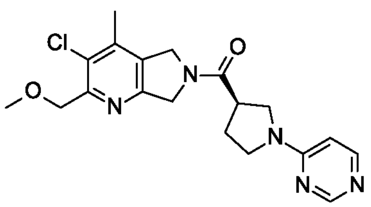

An aromatic nucleophilic substitution reaction using the general procedure as described above, between [3-chloro-2-(methoxymethyl)-4-methyl-5,7-dihydropyrrolo[3,4-b]pyridin-6-yl]-[(3R)-pyrrolidin-3-yl]methanone, dihydrochlorid salt **1-2** and 4-bromopyrimidine, afforded the title product (42% yield) as white solid, MS (ES+) *m/z:* 388.1 [(M+H)$^+$].

**Example 56**

**[3-chloro-2-(methoxymethyl)-4-methyl-5,7-dihydropyrrolo[3,4-b]pyridin-6-yl]-[(3R)-1-(3-pyridyl)pyrrolidin-3-yl] methanone**

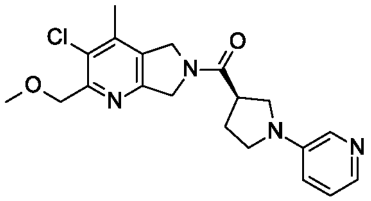

A Buchwald type coupling using the general procedure as described above, between [3-chloro-2-(methoxymethyl)-4-methyl-5,7-dihydropyrrolo[3,4-b]pyridin-6-yl]-[(3R)-pyrrolidin-3-yl]methanone, dihydrochlorid salt **1-2** and 3-bromopyridine, afforded the title product (14% yield) as white solid, MS (ES+) *m/z:* 387.3 [(M+H)$^+$].

**Example 57**

**[3-chloro-2-(methoxymethyl)-4-methyl-5,7-dihydropyrrolo[3,4-b]pyridin-6-yl]-[(3R)-1-[2-(difluoromethyl)-4-pyridyl]pyrrolidin-3-yl]methanone**

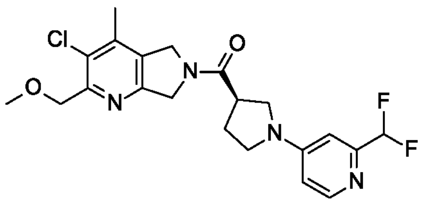

A Buchwald type coupling using the general procedure as described above, between [3-chloro-2-(methoxymethyl)-4-methyl-5,7-dihydropyrrolo[3,4-b]pyridin-6-yl]-[(3R)-pyrrolidin-3-yl]methanone, dihydrochlorid salt 1-2 and 4-bromo-2-(difluoromethyl)pyridine, afforded the title product (15% yield) as white solid, MS (ES+) *m/z:* 437.3 [(M+H)$^+$].

**Example 58**

**[3-chloro-2-(methoxymethyl)-4-methyl-5,7-dihydropyrrolo[3,4-b]pyridin-6-yl]-[(3R)-1-pyrimidin-5-ylpyrrolidin-3-yl]methanone**

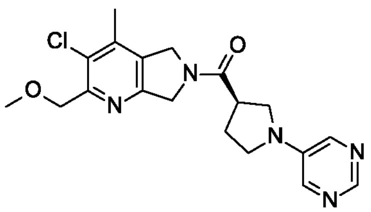

A Buchwald type coupling using the general procedure as described above, between [3-chloro-2-(methoxymethyl)-4-methyl-5,7-dihydropyrrolo[3,4-b]pyridin-6-yl]-[(3R)-pyrrolidin-3-yl]methanone, dihydrochlorid salt **1-2** and 5-bromopyrimidine, afforded the title product (14% yield) as white solid, MS (ES+) *m/z:* 388.3 [(M+H)$^+$].

**Example 59**

**[3-chloro-2-(methoxymethyl)-4-methyl-5,7-dihydropyrrolo[3,4-b]pyridin-6-yl]-[(3R)-1-pyridazin-4-ylpyrrolidin-3-yl] methanone**

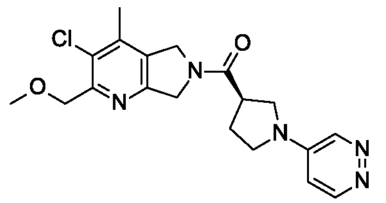

A Buchwald type coupling using the general procedure as described above, between [3-chloro-2-(methoxymethyl)-4-methyl-5,7-dihydropyrrolo[3,4-b]pyridin-6-yl]-[(3R)-pyrrolidin-3-yl]methanone, dihydrochlorid salt **1-2** and 4-bromopyridazine, afforded the title product (20% yield) as white solid, MS (ES+) *m/z:* 388.1 [$(M+H)^+$].

**Example P55**

**1-[3-chloro-2-(methoxymethyl)-4-methyl-5,7-dihydropyrrolo[3,4-b]pyridin-6-yl]-2-(1-pyrimidin-4-ylazetidin-3-yl) ethanone**

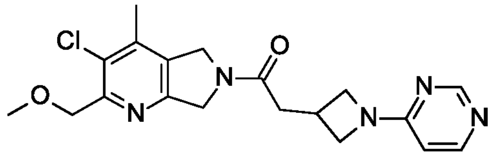

Reference compound P55 was prepared via an amide coupling between 20 mg of 3-chloro-2-(methoxymethyl)-4-methyl-6,7-dihydro-5H-pyrrolo[3,4-b]pyridine dihydrochloride (preparation described in WO2018/002760) and 16 mg (1.2 eq.) of 2-(1-pyrimidin-4-ylazetidin-3-yl)acetic acid (preparation described in WO2018/002760) using TBTU and DIPEA in DMF (1.5 mL) at RT for 3 hours. Purification by column chromatography afforded P55 (24 mg, 88 %) as a white solid. MS (ES+) *m/z:* 388.3 [$(M+H)^+$].

2) Biological examples

2.1) Assay procedures

• Cell culture and stable clone production

CHO cells were transfected with expression plasmid encoding the human M4 receptor and a Gqi5 construct to redirect Gi signaling to calcium. Stable cells were cloned by limiting dilution to yield a monoclonal cell line expressing human M4+Gqi5 (clone 2). The stable cells were grown in DMEM containing 10% FBS, 200 ug/ml Geneticin and 800 ug/ml Hygromycin at 37°C in a 10% $CO_2$ incubator at 95% humidity.

• Calcium flux assay using fluorescent imaging ("M4 PAM FLIPR" assay)

On the afternoon before the assay, cells were plated at a density of 50,000 cells/well into black 96 well plates with clear bottoms to yield a confluent monolayer the next day. Hanks balanced salt solution, without phenol red, containing 20 mM HEPES (pH 7.3) and 2.5 mM probenecid (assay buffer) was prepared fresh for each experiment. Compound dilutions were made using a Beckman Biomek 2000 laboratory automation workstation, in assay buffer. The dye-loading buffer consisted of a final concentration of 2 μM Fluo-4-AM (dissolved in DMSO and pluronic acid) and 65 μg/ml amidoblack in assay buffer. The existing culture media was removed from the wells and 100 μl of the dye-loading buffer was added to each well and incubated for approximately 90 min at 37°C in a 5% $CO_2$ incubator at 95% humidity.

Calcium flux in the dye-loaded cells was measured with the fluorescence reader Hamamatsu FDSS 7000. To test compounds as agonists, compound addition (25 μl) was made 10 s into the fluorescent measurements and fluorescent response was recorded for 3 minutes. The fluorescence data was expressed as percentage of the fluorescence obtained by an EC100 concentration (3 μM) of the endogenous full agonist acetylcholine. Each test compound concentration-response curve was constructed using a four parameter logistic equation in Microsoft Excel XLFit as follows:

$$Y = \text{Minimum} + ((\text{Maximum} - \text{Minimum}) / (1 + 10^{(\text{LogEC50-X})\text{nH})}).$$

The concentration of agonist that produced a half-maximal response is represented by the $EC_{50}$ value. Maximum and minimum are the maximum and minimum fluorescence signal in % respectively. nH is the Hill slope.

To measure positive allosteric modulator activity 25 µl of an $EC_{20}$ concentration of the endogenous agonist acetylcholine is added to the cells already pre-incubated with the test compound for 15 minutes.

The M4 PAM dose response is constructed using the agonist dose-response equation above. The concentration of PAM that produced a half-maximal response is represented by the $EC_{50}$ value and the maximum signal measured is the PAM efficacy expressed in % of the $EC_{100}$ (3 µM) acetylcholine response.

• cAMP Assay for M4 Positive Allosteric Modulators ("M4 PAM cAMP" assay)

The potency of M4 PAMs was determined using a cell-based cyclic adenosine monophosphate assay. The assay was performed with CHO-K1 cells stably expressing untagged human M4 receptor and cAMP was quantified using the Nano-TRF Detection Assay kit (Roche, Cat. No. 05214386) as described in the kit instructions. The cells were grown in Ham's F-12 Nutrient mix supplemented with 10% FBS and 200 µg/ml geneticin until they reached 80% confluency. The cells were washed with PBS, detached with accutase and resuspended in assay buffer consisting of HBSS and 0.1% BSA. They were then seeded in black 384-well plates (Corning, Cat. No. 3764) at a density of 20,000 cells/20µl per well until the addition of compounds.

Test compounds were serially diluted 1:2 in 100% DMSO and spotted in 384-well plates at 0.5 µl/well. The compounds were then diluted in water containing IBMX (Sigma, Cat. No. I7018) and a sample (5 µl) was added to the cells for 15 minutes at 37°C / 5% $CO_2$. After this incubation the cells were stimulated with a combination of forskolin (7.5 µM) and acetylcholine $EC_{20}$ (0.2 nM) in assay buffer for 30 minutes at 37°C / 5% $CO_2$. The assay was stopped by adding cAMP detection mix (15 µl/well, containing detergents for cell lysis) for one hour at RT.

The test plates were read using a SpectraMax i3x reader (Molecular Devices). The exported raw data was used to calculate the FRET signal based on the P-factor as per cAMP kit instructions. The data was normalized to the activity of acetylcholine plus forskolin, with 2 nM of acetylcholine plus 7.5 µM forskolin defined as 100% activity. Dose response curves were fitted to the percent activity data of the compounds using a sigmoidal dose response model (XLfit, IDBS) and the data were reported as $EC_{50}$ and percent efficacy.

• Determination of the in vitro clearance

Incubations of a test compound at different concentrations were performed in 96 well plates containing liver cells (5% $CO_2$ atmosphere and 37°C). At defined time points, either cell medium was taken or the whole well was quenched with acetonitrile containing an internal standard. Samples are then cooled and centrifuged before analysis by LC-MS/MS. Different enzyme markers (Cyp3A4 substrates Midazolam, Quinidin, CYP2B6 Buproprion, CYP2D6 Dextromethorphan, CYP2C9 Tolbutamide, FMO substrate Benzydamine, UGT substrate Raloxifene) for assessing the metabolic activity of the hepatocyte cultures were used as quality control.

Log peak area ratios (test compound peak area / internal standard peak area) or concentrations were plotted against incubation time and a linear fit made to the data with emphasis upon the initial rate of compound disappearance. The slope of the fit is then used to calculate the intrinsic clearance:

$$Cl_{int}\ (\mu L/min/1x10^6\ cells) = -slope\ (min^{-1}) * 1000 / [1x10^6\ cells].$$

Additionally, the intrinsic clearance can also be deduced by the measured rates of metabolite formation.

2.2) Results

Table 1 below shows the data measured in the cAMP and FLIPR assays for all compounds.

Table 1

| Example | M4 PAM cAMP $EC_{50}$ (µM) | M4 PAM FLIPR $EC_{50}$ (µM) |
|---|---|---|
| **1** | 0.155 | 0.387 |
| **2** | 0.079 | 0.061 |

(continued)

| Example | M4 PAM cAMP $EC_{50}$ (μM) | M4 PAM FLIPR $EC_{50}$ (μM) |
|---|---|---|
| **3** | 0.769 | 0.489 |
| **4** | 0.690 | 0.547 |
| **5** | 0.616 | 0.490 |
| **6** | 3.781 | 0.931 |
| **7** | 2.081 | 1.014 |
| **8** | 5.270 | 2.346 |
| **9** | 1.071 | 1.362 |
| **10** | 1.008 | 1.028 |
| **11** | 0.446 | 1.362 |
| **12** | 1.261 | 1.304 |
| **13** | 0.674 | 0.528 |
| **14** | 2.176 | 3.517 |
| **15** | 0.346 | 0.306 |
| **16** | 3.636 | 0.816 |
| **17** | 0.230 | 0.240 |
| **18** | 0.166 | 0.549 |
| **19** | 0.379 | 0.442 |
| **20** | 0.080 | 0.181 |
| **21** | 0.131 | 0.550 |
| **22** | 0.507 | 0.677 |
| **23** | 0.791 | 0.907 |
| **24** | 0.164 | 0.235 |
| **25** | 4.765 | 1.604 |
| **26** | 0.120 | 0.166 |
| **27** | 0.230 | 0.330 |
| **28** | 0.259 | 0.350 |
| **29** | 0.951 | 0.591 |
| **30** | 0.065 | 0.059 |
| **31** | 0.482 | 0.388 |
| **32** | 1.945 | 1.950 |
| **33** | 0.242 | 2.370 |
| **34** | 0.603 | 0.575 |
| **35** | 0.817 | 1.060 |
| **36** | 1.718 | 1.797 |
| **37** | 0.405 | 0.394 |
| **38** | 0.332 | 0.274 |
| **39** | 2.204 | 2.519 |
| **40** | 1.992 | 1.292 |
| **41** | 5.116 | 2.009 |

(continued)

| Example | M4 PAM cAMP $EC_{50}$ (μM) | M4 PAM FLIPR $EC_{50}$ (μM) |
|---|---|---|
| **42** | 2.076 | 0.577 |
| **43** | 7.286 | 1.208 |
| **44** | 1.278 | 1.113 |
| **45** | 1.369 | 0.804 |
| **46** | 0.814 | 0.611 |
| **47** | 1.823 | 0.906 |
| **48** | 8.135 | 2.202 |
| **49** | 3.345 | 1.673 |
| **50** | 4.383 | 1.825 |
| **51** | 0.591 | 0.363 |
| **52** | 1.157 | 0.527 |
| **53** | 0.503 | 0.184 |
| **54** | 0.444 | 0.247 |
| **55** | 0.192 | 0.042 |
| **56** | 0.080 | 0.486 |
| **57** | 0.283 | 0.099 |
| **58** | 0.179 | 0.074 |
| **59** | 0.881 | 0.209 |

WO 2018/002760 discloses reference compounds P1 as example 13 and P2 as example 39. Reference compound P55 has been prepared as described herein.

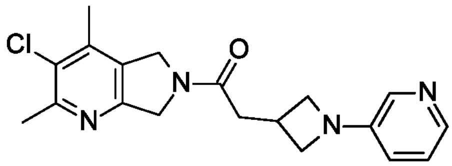

P1

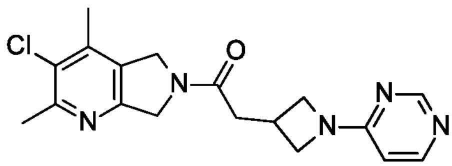

P2

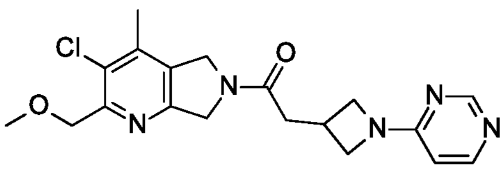

P55

Affinity of the reference compounds towards the M4 receptor, as well as in vitro clearance of the reference compounds and examples 1, 2 and 55 were also determined. The results are shown in table 2.

Table 2

| Example | M4 PAM cAMP $EC_{50}$ (μM) | M4 PAM FLIPR $EC_{50}$ (μM) | Clearance (μl/min/1*$10^6$ cells) |
|---|---|---|---|
| 1 | 0.155 | 0.387 | 9.3 |
| P1 | 0.055 | 0.947 | 20 |
| 2 | 0.079 | 0.061 | 3.5 |
| P2 | 0.053 | 0.035 | 7.9 |
| 55 | 0.192 | 0.042 | 2.1 |
| P55 | / | 0.050 | 5.1 |

Examples 1, 2 and 55 exhibit clearance values reduced by 50% compared to clearance values of examples P1, P2 and P55.

## Claims

1. A compound of formula (I)

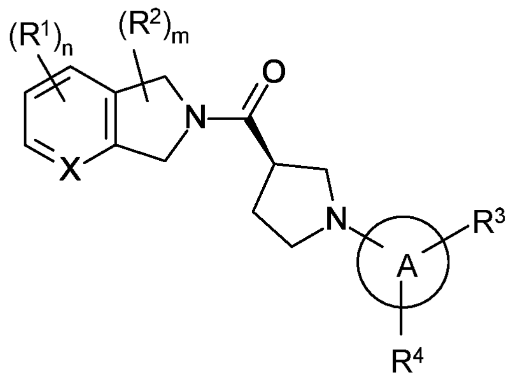

(I)

or a pharmaceutically acceptable salt thereof, wherein:

A is a 6-membered heteroaryl group selected from pyridinyl, pyradizinyl, pyrazinyl and pyrimidinyl;
$R^1$ is halogen, $C_1$-$C_6$-alkyl or $C_2$-$C_8$-alkoxyalkyl;
$R^3$ and $R^4$ are each independently selected from hydrogen, $C_1$-$C_6$-alkyl, $C_1$-$C_6$-haloalkyl, $C_1$-$C_6$- alkoxy, $C_1$-$C_6$-haloalkoxy, cyano, $C_3$-$C_{12}$-cycloalkyl, $C_2$-$C_9$-heterocycloalkyl, -$NR^5R^6$ and -$S(O)_2$-$R^7$;
$R^5$ and $R^6$ are each independently selected from hydrogen and $C_1$-$C_6$-alkyl, and $R^7$ is $C_1$-$C_6$-alkyl;
X is N;
n is 3; and
m is 0.

2. The compound of formula (I) according to claim 1, or a pharmaceutically acceptable salt thereof, wherein A is pyridinyl or pyrimidinyl.

3. The compound of formula (I) according to any one of claims 1 or 2, or a pharmaceutically acceptable salt thereof, wherein $R^1$ is chlorine, methyl or methoxymethyl.

4. The compound of formula (I) according to any one of claims 1 to 3, or a pharmaceutically acceptable salt thereof, wherein $R^3$ and $R^4$ are each independently selected from hydrogen, $C_1$-$C_6$-haloalkyl, $C_1$-$C_6$-haloalkoxy and cyano.

5. The compound of formula (I) according to any one of claims 1 to 4, or a pharmaceutically acceptable salt thereof,

wherein $R^3$ and $R^4$ are each independently selected from hydrogen, $-CHF_2$, $-CF_3$, $-OCHF_2$ and cyano.

**6.** The compound of formula (I) according to claim 1, or a pharmaceutically acceptable salt thereof, wherein:

A is pyridinyl or pyrimidinyl;
$R^1$ is halogen, $C_1$-$C_6$-alkyl or $C_2$-$C_8$-alkoxyalkyl;
$R^3$ and $R^4$ are each independently selected from hydrogen, $C_1$-$C_6$-haloalkyl, $C_1$-$C_6$-haloalkoxy and cyano;
X is N;
n is 3; and
m is 0.

**7.** The compound of formula (I) according to claim 1, or a pharmaceutically acceptable salt thereof, wherein:

A is pyridinyl or pyrimidinyl;
$R^1$ is chlorine, methyl or methoxymethyl;
$R^3$ and $R^4$ are each independently selected from hydrogen, $-CHF_2$, $-CF_3$, $-OCHF_2$ and cyano;
X is N;
n is 3; and
m is 0.

**8.** The compound of formula (I) according to claim 1, or a pharmaceutically acceptable salt thereof, selected from:

(3-chloro-2,4-dimethyl-5,7-dihydropyrrolo[3,4-b]pyridin-6-yl)-[(3R)-1-(3-pyridyl)pyrrolidin-3-yl]methanone;
(3-chloro-2,4-dimethyl-5,7-dihydropyrrolo[3,4-b]pyridin-6-yl)-[(3R)-1-pyrimidin-4-ylpyrrolidin-3-yl]methanone;
(3-chloro-2,4-dimethyl-5,7-dihydropyrrolo[3,4-b]pyridin-6-yl)-[(3R)-1-(4-pyridyl)pyrrolidin-3-yl]methanone;
(3-chloro-2,4-dimethyl-5,7-dihydropyrrolo[3,4-b]pyridin-6-yl)-[(3R)-1-(6-methyl-3-pyridyl)pyrrolidin-3-yl]methanone;
(3-chloro-2,4-dimethyl-5,7-dihydropyrrolo[3,4-b]pyridin-6-yl)-[(3R)-1-(2-methyl-4-pyridyl)pyrrolidin-3-yl]methanone;
(3-chloro-2,4-dimethyl-5,7-dihydropyrrolo[3,4-b]pyridin-6-yl)-[(3R)-1-(2-cyclopropylpyrimidin-4-yl)pyrrolidin-3-yl]methanone;
(3-chloro-2,4-dimethyl-5,7-dihydropyrrolo[3,4-b]pyridin-6-yl)-[(3R)-1-[5-(trifluoromethyl)pyrimidin-2-yl]pyrrolidin-3-yl]methanone;
(3-chloro-2,4-dimethyl-5,7-dihydropyrrolo[3,4-b]pyridin-6-yl)-[(3R)-1-(5-methylpyrimidin-2-yl)pyrrolidin-3-yl]methanone;
(3-chloro-2,4-dimethyl-5,7-dihydropyrrolo[3,4-b]pyridin-6-yl)-[(3R)-1-[6-(trifluoromethyl)-3-pyridyl]pyrrolidin-3-yl]methanone;
(3-chloro-2,4-dimethyl-5,7-dihydropyrrolo[3,4-b]pyridin-6-yl)-[(3R)-1-(6-methoxy-3-pyridyl)pyrrolidin-3-yl]methanone;
(3-chloro-2,4-dimethyl-5,7-dihydropyrrolo[3,4-b]pyridin-6-yl)-[(3R)-1-[6-(methylamino)-3-pyridyl]pyrrolidin-3-yl]methanone;
(3-chloro-2,4-dimethyl-5,7-dihydropyrrolo[3,4-b]pyridin-6-yl)-[(3R)-1-(2-methyl-3-pyridyl)pyrrolidin-3-yl]methanone;
(3-chloro-2,4-dimethyl-5,7-dihydropyrrolo[3,4-b]pyridin-6-yl)-[(3R)-1-(4-methyl-3-pyridyl)pyrrolidin-3-yl]methanone;
(3-chloro-2,4-dimethyl-5,7-dihydropyrrolo[3,4-b]pyridin-6-yl)-[(3R)-1-[5-(trifluoromethyl)-3-pyridyl]pyrrolidin-3-yl]methanone;
(3-chloro-2,4-dimethyl-5,7-dihydropyrrolo[3,4-b]pyridin-6-yl)-[(3R)-1-[5-(difluoromethyl)-3-pyridyl]pyrrolidin-3-yl]methanone;
(3-chloro-2,4-dimethyl-5,7-dihydropyrrolo[3,4-b]pyridin-6-yl)-[(3R)-1-(5-cyclopropyl-3-pyridyl)pyrrolidin-3-yl]methanone;
(3-chloro-2,4-dimethyl-5,7-dihydropyrrolo[3,4-b]pyridin-6-yl)-[(3R)-1-[5-(difluoromethoxy)-3-pyridyl]pyrrolidin-3-yl]methanone;
(3-chloro-2,4-dimethyl-5,7-dihydropyrrolo[3,4-b]pyridin-6-yl)-[(3R)-1-(5-methoxy-3-pyridyl)pyrrolidin-3-yl]methanone;
(3-chloro-2,4-dimethyl-5,7-dihydropyrrolo[3,4-b]pyridin-6-yl)-[(3R)-1-(5-methylsulfonyl-3-pyridyl)pyrrolidin-3-yl]methanone;
(3-chloro-2,4-dimethyl-5,7-dihydropyrrolo[3,4-b]pyridin-6-yl)-[(3R)-1-[2-(difluoromethyl)-4-pyridyl]pyrrolidin-3-

yl]methanone;
(3-chloro-2,4-dimethyl-5,7-dihydropyrrolo[3,4-b]pyridin-6-yl)-[(3R)-1-[2-(trifluoromethyl)-4-pyridyl]pyrrolidin-3-yl]methanone;
(3-chloro-2,4-dimethyl-5,7-dihydropyrrolo[3,4-b]pyridin-6-yl)-[(3R)-1-(2-methoxy-4-pyridyl)pyrrolidin-3-yl]methanone;
(3-chloro-2,4-dimethyl-5,7-dihydropyrrolo[3,4-b]pyridin-6-yl)-[(3R)-1-[2-(trifluoromethyl)pyrimidin-4-yl]pyrrolidin-3-yl]methanone;
4-[(3R)-3-(3-chloro-2,4-dimethyl-5,7-dihydropyrrolo[3,4-b]pyridine-6-carbonyl)pyrrolidin-1-yl]pyrimidine-2-carbonitrile;
(3-chloro-2,4-dimethyl-5,7-dihydropyrrolo[3,4-b]pyridin-6-yl)-[(3R)-1-(2-methoxypyrimidin-4-yl)pyrrolidin-3-yl]methanone;
(3-chloro-2,4-dimethyl-5,7-dihydropyrrolo[3,4-b]pyridin-6-yl)-[(3R)-1-(6-methylpyrimidin-4-yl)pyrrolidin-3-yl]methanone;
(3-chloro-2,4-dimethyl-5,7-dihydropyrrolo[3,4-b]pyridin-6-yl)-[(3R)-1-[6-(trifluoromethyl)pyrimidin-4-yl]pyrrolidin-3-yl]methanone;
(3-chloro-2,4-dimethyl-5,7-dihydropyrrolo[3,4-b]pyridin-6-yl)-[(3R)-1-(6-cyclopropylpyrimidin-4-yl)pyrrolidin-3-yl]methanone;
(3-chloro-2,4-dimethyl-5,7-dihydropyrrolo[3,4-b]pyridin-6-yl)-[(3R)-1-(6-methoxypyrimidin-4-yl)pyrrolidin-3-yl]methanone;
(3-chloro-2,4-dimethyl-5,7-dihydropyrrolo[3,4-b]pyridin-6-yl)-[(3R)-1-pyrimidin-5-ylpyrrolidin-3-yl]methanone;
(3-chloro-2,4-dimethyl-5,7-dihydropyrrolo[3,4-b]pyridin-6-yl)-[(3R)-1-(2-methylpyrimidin-5-yl)pyrrolidin-3-yl]methanone;
(3-chloro-2,4-dimethyl-5,7-dihydropyrrolo[3,4-b]pyridin-6-yl)-[(3R)-1-[2-(trifluoromethyl)pyrimidin-5-yl]pyrrolidin-3-yl]methanone;
(3-chloro-2,4-dimethyl-5,7-dihydropyrrolo[3,4-b]pyridin-6-yl)-[(3R)-1-(2-methoxypyrimidin-5-yl)pyrrolidin-3-yl]methanone;
(3-chloro-2,4-dimethyl-5,7-dihydropyrrolo[3,4-b]pyridin-6-yl)-[(3R)-1-[2-(oxetan-3-yl)pyrimidin-5-yl]pyrrolidin-3-yl]methanone;
(3-chloro-2,4-dimethyl-5,7-dihydropyrrolo[3,4-b]pyridin-6-yl)-[(3R)-1-[2-(methylamino)pyrimidin-5-yl]pyrrolidin-3-yl]methanone;
(3-chloro-2,4-dimethyl-5,7-dihydropyrrolo[3,4-b]pyridin-6-yl)-[(3R)-1-[2-(dimethylamino)pyrimidin-5-yl]pyrrolidin-3-yl]methanone;
(3-chloro-2,4-dimethyl-5,7-dihydropyrrolo[3,4-b]pyridin-6-yl)-[(3R)-1-(4-methylpyrimidin-5-yl)pyrrolidin-3-yl]methanone;
(3-chloro-2,4-dimethyl-5,7-dihydropyrrolo[3,4-b]pyridin-6-yl)-[(3R)-1-pyridazin-4-ylpyrrolidin-3-yl]methanone;
(3-chloro-2,4-dimethyl-5,7-dihydropyrrolo[3,4-b]pyridin-6-yl)-[(3R)-1-pyridazin-3-ylpyrrolidin-3-yl]methanone;
(3-chloro-2,4-dimethyl-5,7-dihydropyrrolo[3,4-b]pyridin-6-yl)-[(3R)-1-(6-methylpyridazin-3-yl)pyrrolidin-3-yl]methanone;
(3-chloro-2,4-dimethyl-5,7-dihydropyrrolo[3,4-b]pyridin-6-yl)-[(3R)-1-[6-(trifluoromethyl)pyridazin-3-yl]pyrrolidin-3-yl]methanone;
(3-chloro-2,4-dimethyl-5,7-dihydropyrrolo[3,4-b]pyridin-6-yl)-[(3R)-1-(6-methoxypyrazin-2-yl)pyrrolidin-3-yl]methanone;
(3-chloro-2,4-dimethyl-5,7-dihydropyrrolo[3,4-b]pyridin-6-yl)-[(3R)-1-(6-cyclopropylpyrazin-2-yl)pyrrolidin-3-yl]methanone;
5-[(3R)-3-(3-chloro-2,4-dimethyl-5,7-dihydropyrrolo[3,4-b]pyridine-6-carbonyl)pyrrolidin-1-yl]pyrazine-2-carbonitrile;
(3-chloro-2,4-dimethyl-5,7-dihydropyrrolo[3,4-b]pyridin-6-yl)-[(3R)-1-(5-methoxypyrazin-2-yl)pyrrolidin-3-yl]methanone;
(3-chloro-2,4-dimethyl-5,7-dihydropyrrolo[3,4-b]pyridin-6-yl)-[(3R)-1-(5-methylpyrazin-2-yl)pyrrolidin-3-yl]methanone;
(3-chloro-2,4-dimethyl-5,7-dihydropyrrolo[3,4-b]pyridin-6-yl)-[(3R)-1-[5-(trifluoromethyl)pyrazin-2-yl]pyrrolidin-3-yl]methanone;
(3-chloro-2,4-dimethyl-5,7-dihydropyrrolo[3,4-b]pyridin-6-yl)-[(3R)-1-(4-methoxypyrimidin-2-yl)pyrrolidin-3-yl]methanone;
(3-chloro-2,4-dimethyl-5,7-dihydropyrrolo[3,4-b]pyridin-6-yl)-[(3R)-1-(4-methylpyrimidin-2-yl)pyrrolidin-3-yl]methanone;
(3-chloro-2,4-dimethyl-5,7-dihydropyrrolo[3,4-b]pyridin-6-yl)-[(3R)-1-[4-(trifluoromethyl)pyrimidin-2-yl]pyrrolidin-3-yl]methanone;

(3-chloro-2,4-dimethyl-5,7-dihydropyrrolo[3,4-b]pyridin-6-yl)-[(3R)-1-(6-methylpyrazin-2-yl)pyrrolidin-3-yl]methanone;
(3-chloro-2,4-dimethyl-5,7-dihydropyrrolo[3,4-b]pyridin-6-yl)-[(3R)-1-(6-methylpyridazin-4-yl)pyrrolidin-3-yl]methanone;
[3-chloro-2-(methoxymethyl)-4-methyl-5,7-dihydropyrrolo[3,4-b]pyridin-6-yl]-[(3R)-1-(6-methylpyrimidin-4-yl)pyrrolidin-3-yl]methanone;
(3-chloro-2,4-dimethyl-5,7-dihydropyrrolo[3,4-b]pyridin-6-yl)-[(3R)-1-[6-(difluoromethyl)pyrimidin-4-yl]pyrrolidin-3-yl]methanone;
[3-chloro-2-(methoxymethyl)-4-methyl-5,7-dihydropyrrolo[3,4-b]pyridin-6-yl]-[(3R)-1-pyrimidin-4-ylpyrrolidin-3-yl]methanone;
[3-chloro-2-(methoxymethyl)-4-methyl-5,7-dihydropyrrolo[3,4-b]pyridin-6-yl]-[(3R)-1-(3-pyridyl)pyrrolidin-3-yl]methanone;
[3-chloro-2-(methoxymethyl)-4-methyl-5,7-dihydropyrrolo[3,4-b]pyridin-6-yl]-[(3R)-1-[2-(difluoromethyl)-4-pyridyl]pyrrolidin-3-ylmethanone;
[3-chloro-2-(methoxymethyl)-4-methyl-5,7-dihydropyrrolo[3,4-b]pyridin-6-yl]-[(3R)-1-pyrimidin-5-ylpyrrolidin-3-yl]methanone; and
[3-chloro-2-(methoxymethyl)-4-methyl-5,7-dihydropyrrolo[3,4-b]pyridin-6-yl]-[(3R)-1-pyridazin-4-ylpyrrolidin-3-yl]methanone.

**9.** A compound of formula (I) according to claim 1, or a pharmaceutically acceptable salt thereof, selected from:

(3-chloro-2,4-dimethyl-5,7-dihydropyrrolo[3,4-b]pyridin-6-yl)-[(3R)-1-(3-pyridyl)pyrrolidin-3-yl]methanone;
(3-chloro-2,4-dimethyl-5,7-dihydropyrrolo[3,4-b]pyridin-6-yl)-[(3R)-1-pyrimidin-4-ylpyrrolidin-3-yl]methanone;
(3-chloro-2,4-dimethyl-5,7-dihydropyrrolo[3,4-b]pyridin-6-yl)-[(3R)-1-[5-(difluoromethoxy)-3-pyridyl]pyrrolidin-3-yl]methanone;
(3-chloro-2,4-dimethyl-5,7-dihydropyrrolo[3,4-b]pyridin-6-yl)-[(3R)-1-[2-(difluoromethyl)-4-pyridyl]pyrrolidin-3-yl]methanone;
(3-chloro-2,4-dimethyl-5,7-dihydropyrrolo[3,4-b]pyridin-6-yl)-[(3R)-1-[2-(trifluoromethyl)-4-pyridyl]pyrrolidin-3-yl]methanone;
4-[(3R)-3-(3-chloro-2,4-dimethyl-5,7-dihydropyrrolo[3,4-b]pyridine-6-carbonyl)pyrrolidin-1-yl]pyrimidine-2-carbonitrile;
(3-chloro-2,4-dimethyl-5,7-dihydropyrrolo[3,4-b]pyridin-6-yl)-[(3R)-1-pyrimidin-5-ylpyrrolidin-3-yl]methanone;
(3-chloro-2,4-dimethyl-5,7-dihydropyrrolo[3,4-b]pyridin-6-yl)-[(3R)-1-[6-(difluoromethyl)pyrimidin-4-yl]pyrrolidin-3-yl]methanone;
3-chloro-2-(methoxymethyl)-4-methyl-5,7-dihydropyrrolo[3,4-b]pyridin-6-yl]-[(3R)-1-pyrimidin-4-ylpyrrolidin-3-yl]methanone; and
[3-chloro-2-(methoxymethyl)-4-methyl-5,7-dihydropyrrolo[3,4-b]pyridin-6-yl]-[(3R)-1-(3-pyridyl)pyrrolidin-3-yl]methanone.

**10.** A process for the preparation of a compound of formula (I) according to any one of claims 1 to 9, or a pharmaceutically acceptable salt thereof, comprising reacting an amine salt 1

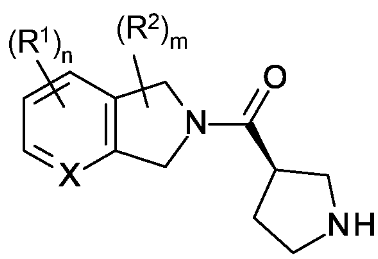

. 2HCl **1**

with a halide compound 2

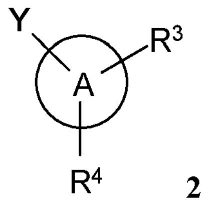

2

wherein $R^1$, $R^2$, $R^3$, $R^4$, X and A are as defined in any one of claims 1 to 9, and Y is a halogen, to form said compound of formula (I), and optionally converting the compounds obtained into a pharmaceutically acceptable salt thereof.

**11.** A compound of formula (I) according to any one of claims 1 to 9, or a pharmaceutically acceptable salt thereof, for use as therapeutically active substance.

**12.** A pharmaceutical composition comprising a compound of formula (I) according to any one of claims 1 to 9, or a pharmaceutically acceptable salt thereof, and a pharmaceutically acceptable excipient.

**13.** A compound of formula (I) according to any one of claims 1 to 9, or a pharmaceutically acceptable salt thereof, for use in the therapeutic and/or prophylactic treatment of Alzheimer's disease, schizophrenia, psychosis, Parkinson's disease, pain, addiction and Huntington's disease.

**Patentansprüche**

**1.** Verbindung der Formel (I)

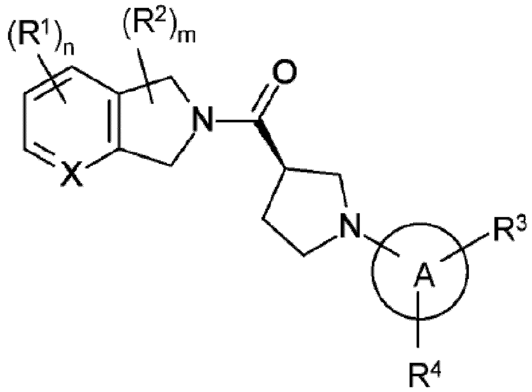

(I)

oder ein pharmazeutisch unbedenkliches Salz davon, wobei:

A eine 6-gliedrige Heteroarylgruppe, ausgewählt aus Pyridinyl, Pyradizinyl, Pyrazinyl und Pyrimidinyl, ist;
$R^1$ Halogen, $C_1$-$C_6$-Alkyl oder $C_2$-$C_8$-Alkoxyalkyl ist;
$R^3$ und $R^4$ jeweils unabhängig voneinander aus Wasserstoff, $C_1$-$C_6$-Alkyl, $C_1$-$C_6$-Halogenalkyl, $C_1$-$C_6$-Alkoxy, $C_1$-$C_6$-Halogenalkoxy, Cyano, $C_3$-$C_{12}$-Cycloalkyl, $C_2$-$C_9$-Heterocycloalkyl, $-NR^5R^6$ und $-S(O)_2-R^7$ ausgewählt sind;
$R^5$ und $R^6$ jeweils unabhängig voneinander aus Wasserstoff und $C_1$-$C_6$-Alkyl ausgewählt sind und $R^7$ $C_1$-$C_6$-Alkyl ist;
X N ist;
n 3 ist und
m 0 ist.

**2.** Verbindung der Formel (I) nach Anspruch 1 oder ein pharmazeutisch unbedenkliches Salz davon, wobei A Pyridinyl oder Pyrimidinyl ist.

**3.** Verbindung der Formel (I) nach einem der Ansprüche 1 oder 2 oder ein pharmazeutisch unbedenkliches Salz davon,

wobei $R^1$ Chlor, Methyl oder Methoxymethyl ist.

4. Verbindung der Formel (I) nach einem der Ansprüche 1 bis 3 oder ein pharmazeutisch unbedenkliches Salz davon, wobei $R^3$ und $R^4$ jeweils unabhängig voneinander aus Wasserstoff, $C_1$-$C_6$-Halogenalkyl, $C_1$-$C_6$-Halogenalkoxy und Cyano ausgewählt sind.

5. Verbindung der Formel (I) nach einem der Ansprüche 1 bis 4 oder ein pharmazeutisch unbedenkliches Salz davon, wobei $R^3$ und $R^4$ jeweils unabhängig voneinander aus Wasserstoff, $-CHF_2$, $-CF_3$, $-OCHF_2$ und Cyano ausgewählt sind.

6. Verbindung der Formel (I) nach Anspruch 1 oder ein pharmazeutisch unbedenkliches Salz davon, wobei:

   A Pyridinyl oder Pyrimidinyl ist;
   $R^1$ Halogen, $C_1$-$C_6$-Alkyl oder $C_2$-$C_8$-Alkoxyalkyl ist;
   $R^3$ und $R^4$ jeweils unabhängig voneinander aus Wasserstoff, $C_1$-$C_6$-Halogenalkyl, $C_1$-$C_6$-Halogenalkoxy und Cyano ausgewählt sind;
   X N ist;
   n 3 ist und
   m 0 ist.

7. Verbindung der Formel (I) nach Anspruch 1 oder ein pharmazeutisch unbedenkliches Salz davon, wobei:

   A Pyridinyl oder Pyrimidinyl ist;
   $R^1$ Chlor, Methyl oder Methoxymethyl ist;
   $R^3$ und $R^4$ jeweils unabhängig voneinander aus Wasserstoff, $-CHF_2$, $-CF_3$, $-OCHF_2$ und Cyano ausgewählt sind;
   X N ist;
   n 3 ist und
   m 0 ist.

8. Verbindung der Formel (I) nach Anspruch 1 oder ein pharmazeutisch unbedenkliches Salz davon, ausgewählt aus:

   (3-Chlor-2,4-dimethyl-5,7-dihydropyrrolo[3,4-b]pyridin-6-yl)-[(3R)-1-(3-pyridyl)pyrrolidin-3-yl]methanon;
   (3-Chlor-2,4-dimethyl-5,7-dihydropyrrolo[3,4-b]pyridin-6-yl)-[(3R)-1-pyrimidin-4-ylpyrroli-din-3-yl]methanon;
   (3-Chlor-2,4-dimethyl-5,7-dihydropyrrolo[3,4-b]pyridin-6-yl)-[(3R)-1-(4-pyridyl)pyrrolidin-3-yl]methanon;
   (3-Chlor-2,4-dimethyl-5,7-dihydropyrrolo[3,4-b]pyridin-6-yl)-[(3R)-1-(6-methyl-3-pyridyl) pyrrolidin-3-yl]methanon;
   (3-Chlor-2,4-dimethyl-5,7-dihydropyrrolo[3,4-b]pyridin-6-yl)-[(3R)-1-(2-methyl-4-pyridyl) pyrrolidin-3-yl]methanon;
   (3-Chlor-2,4-dimethyl-5,7-dihydropyrrolo[3,4-b]pyridin-6-yl)-[(3R)-1-(2-cyclopropylpyrimi-din-4-yl)pyrrolidin-3-yl]methanon;
   (3-Chlor-2,4-dimethyl-5,7-dihydropyrrolo[3,4-b]pyridin-6-yl)-[(3R)-1-[5-(trifluormethyl)pyrimidin-2-yl]pyrrolidin-3-yl]methanon;
   (3-Chlor-2,4-dimethyl-5,7-dihydropyrrolo[3,4-b]pyridin-6-yl)-[(3R)-1-(5-methylpyrimidin-2-yl)pyrrolidin-3-yl]methanon;
   (3-Chlor-2,4-dimethyl-5,7-dihydropyrrolo[3,4-b]pyridin-6-yl)-[(3R)-1-[6-(trifluormethyl)-3-pyridyl]pyrrolidin-3-yl]methanon;
   (3-Chlor-2,4-dimethyl-5,7-dihydropyrrolo[3,4-b]pyridin-6-yl)-[(3R)-1-(6-methoxy-3-pyridyl) pyrrolidin-3-yl]methanon;
   (3-Chlor-2,4-dimethyl-5,7-dihydropyrrolo[3,4-b]pyridin-6-yl)-[(3R)-1-[6-(methylamino)-3-pyridyl]pyrrolidin-3-yl]methanon;
   (3-Chlor-2,4-dimethyl-5,7-dihydropyrrolo[3,4-b]pyridin-6-yl)-[(3R)-1-(2-methyl-3-pyridyl) pyrrolidin-3-yl]methanon;
   (3-Chlor-2,4-dimethyl-5,7-dihydropyrrolo[3,4-b]pyridin-6-yl)-[(3R)-1-(4-methyl-3-pyridyl) pyrrolidin-3-yl]methanon;
   (3-Chlor-2,4-dimethyl-5,7-dihydropyrrolo[3,4-b]pyridin-6-yl)-[(3R)-1-[5-(trifluormethyl)-3-pyridyl]pyrrolidin-3-yl]methanon;
   (3-Chlor-2,4-dimethyl-5,7-dihydropyrrolo[3,4-b]pyridin-6-yl)-[(3R)-1-[5-(difluormethyl)-3-pyridyl]pyrrolidin-3-yl]methanon;

(3-Chlor-2,4-dimethyl-5,7-dihydropyrrolo[3,4-b]pyridin-6-yl)-[(3R)-1-(5-cyclopropyl-3-pyridyl)pyrrolidin-3-yl]methanon;
(3-Chlor-2,4-dimethyl-5,7-dihydropyrrolo[3,4-b]pyridin-6-yl)-[(3R)-1-[5-(difluormethoxy)-3-pyridyl]pyrrolidin-3-yl]methanon;
(3-Chlor-2,4-dimethyl-5,7-dihydropyrrolo[3,4-b]pyridin-6-yl)-[(3R)-1-(5-methoxy-3-pyridyl) pyrrolidin-3-yl]methanon;
(3-Chlor-2,4-dimethyl-5,7-dihydropyrrolo[3,4-b]pyridin-6-yl)-[(3R)-1-(5-methylsulfonyl-3-pyridyl)pyrrolidin-3-yl]methanon;
(3-Chlor-2,4-dimethyl-5,7-dihydropyrrolo[3,4-b]pyridin-6-yl)-[(3R)-1-[2-(difluormethyl)-4-pyridyl]pyrrolidin-3-yl]methanon;
(3-Chlor-2,4-dimethyl-5,7-dihydropyrrolo[3,4-b]pyridin-6-yl)-[(3R)-1-[2-(trifluormethyl)-4-pyridyl]pyrrolidin-3-yl]methanon;
(3-Chlor-2,4-dimethyl-5,7-dihydropyrrolo[3,4-b]pyridin-6-yl)-[(3R)-1-(2-methoxy-4-pyridyl) pyrrolidin-3-yl]methanon;
(3-Chlor-2,4-dimethyl-5,7-dihydropyrrolo[3,4-b]pyridin-6-yl)-[(3R)-1-[2-(trifluormethyl)pyrimidin-4-yl]pyrrolidin-3-yl]methanon;
4-[(3R)-3-(3-Chlor-2,4-dimethyl-5,7-dihydropyrrolo[3,4-b]pyridin-6-carbonyl)pyrrolidin-1-yl]pyrimidin-2-carbonitril;
(3-Chlor-2,4-dimethyl-5,7-dihydropyrrolo[3,4-b]pyridin-6-yl)-[(3R)-1-(2-methoxypyrimidin-4-yl)pyrrolidin-3-yl]methanon;
(3-Chlor-2,4-dimethyl-5,7-dihydropyrrolo[3,4-b]pyridin-6-yl)-[(3R)-1-(6-methylpyrimidin-4-yl)pyrrolidin-3-yl]methanon;
(3-Chlor-2,4-dimethyl-5,7-dihydropyrrolo[3,4-b]pyridin-6-yl)-[(3R)-1-[6-(trifluormethyl)pyrimidin-4-yl]pyrrolidin-3-yl]methanon;
(3-Chlor-2,4-dimethyl-5,7-dihydropyrrolo[3,4-b]pyridin-6-yl)-[(3R)-1-(6-cyclopropylpyrimi-din-4-yl)pyrrolidin-3-yl]methanon;
(3-Chlor-2,4-dimethyl-5,7-dihydropyrrolo[3,4-b]pyridin-6-yl)-[(3R)-1-(6-methoxypyrimidin-4-yl)pyrrolidin-3-yl]methanon;
(3-Chlor-2,4-dimethyl-5,7-dihydropyrrolo[3,4-b]pyridin-6-yl)-[(3R)-1-pyrimidin-5-ylpyrroli-din-3-yl]methanon;
(3-Chlor-2,4-dimethyl-5,7-dihydropyrrolo[3,4-b]pyridin-6-yl)-[(3R)-1-(2-methylpyrimidin-5-yl)pyrrolidin-3-yl]methanon;
(3-Chlor-2,4-dimethyl-5,7-dihydropyrrolo[3,4-b]pyridin-6-yl)-[(3R)-1-[2-(trifluormethyl)pyrimidin-5-yl]pyrrolidin-3-yl]methanon;
(3-Chlor-2,4-dimethyl-5,7-dihydropyrrolo[3,4-b]pyridin-6-yl)-[(3R)-1-(2-methoxypyrimidin-5-yl)pyrrolidin-3-yl]methanon;
(3-Chlor-2,4-dimethyl-5,7-dihydropyrrolo[3,4-b]pyridin-6-yl)-[(3R)-1-[2-(oxetan-3-yl)pyrimidin-5-yl]pyrrolidin-3-yl]methanon;
(3-Chlor-2,4-dimethyl-5,7-dihydropyrrolo[3,4-b]pyridin-6-yl)-[(3R)-1-[2-(methylamino)pyrimidin-5-yl]pyrrolidin-3-yl]methanon;
(3-Chlor-2,4-dimethyl-5,7-dihydropyrrolo[3,4-b]pyridin-6-yl)-[(3R)-1-[2-(dimethylamino)pyrimidin-5-yl]pyrrolidin-3-yl]methanon;
(3-Chlor-2,4-dimethyl-5,7-dihydropyrrolo[3,4-b]pyridin-6-yl)-[(3R)-1-(4-methylpyrimidin-5-yl)pyrrolidin-3-yl]methanon;
(3-Chlor-2,4-dimethyl-5,7-dihydropyrrolo[3,4-b]pyridin-6-yl)-[(3R)-1-pyridazin-4-ylpyrroli-din-3-yl]methanon;
(3-Chlor-2,4-dimethyl-5,7-dihydropyrrolo[3,4-b]pyridin-6-yl)-[(3R)-1-pyridazin-3-ylpyrroli-din-3-yl]methanon;
(3-Chlor-2,4-dimethyl-5,7-dihydropyrrolo[3,4-b]pyridin-6-yl)-[(3R)-1-(6-methylpyridazin-3-yl)pyrrolidin-3-yl]methanon;
(3-Chlor-2,4-dimethyl-5,7-dihydropyrrolo[3,4-b]pyridin-6-yl)-[(3R)-1-[6-(trifluormethyl)pyridazin-3-yl]pyrrolidin-3-yl]methanon;
(3-Chlor-2,4-dimethyl-5,7-dihydropyrrolo[3,4-b]pyridin-6-yl)-[(3R)-1-(6-methoxypyrazin-2-yl)pyrrolidin-3-yl]methanon;
(3-Chlor-2,4-dimethyl-5,7-dihydropyrrolo[3,4-b]pyridin-6-yl)-[(3R)-1-(6-cyclopropylpyrazin-2-yl)pyrrolidin-3-yl]methanon;
5-[(3R)-3-(3-Chlor-2,4-dimethyl-5,7-dihydropyrrolo[3,4-b]pyridin-6-carbonyl)pyrrolidin-1-yl]pyrazin-2-carbonitril;
(3-Chlor-2,4-dimethyl-5,7-dihydropyrrolo[3,4-b]pyridin-6-yl)-[(3R)-1-(5-methoxypyrazin-2-yl)pyrrolidin-3-yl]methanon;
(3-Chlor-2,4-dimethyl-5,7-dihydropyrrolo[3,4-b]pyridin-6-yl)-[(3R)-1-(5-methylpyrazin-2-yl) pyrrolidin-3-yl]methanon
(3-Chlor-2,4-dimethyl-5,7-dihydropyrrolo[3,4-b]pyridin-6-yl)-[(3R)-1-[5-(trifluormethyl)pyrazin-2-yl]pyrrolidin-3-yl]methanon;
(3-Chlor-2,4-dimethyl-5,7-dihydropyrrolo[3,4-b]pyridin-6-yl)-[(3R)-1-(4-methoxypyrimidin-2-yl)pyrrolidin-3-yl] methanon;
(3-Chlor-2,4-dimethyl-5,7-dihydropyrrolo[3,4-b]pyridin-6-yl)-[(3R)-1-(4-methylpyrimidin-2-yl)pyrrolidin-3-yl]methanon;
(3-Chlor-2,4-dimethyl-5,7-dihydropyrrolo[3,4-b]pyridin-6-yl)-[(3R)-1-[4-(trifluormethyl)pyrimidin-2-yl]pyrrolidin-3-yl]methanon;
(3-Chlor-2,4-dimethyl-5,7-dihydropyrrolo[3,4-b]pyridin-6-yl)-[(3R)-1-(6-methylpyrazin-2-yl) pyrrolidin-3-yl]methanon;
(3-Chlor-2,4-dimethyl-5,7-dihydropyrrolo[3,4-b]pyridin-6-yl)-[(3R)-1-(6-methylpyridazin-4-yl)pyrrolidin-3-yl]methanon;
[3-Chlor-2-(methoxymethyl)-4-methyl-5,7-dihydropyrrolo[3,4-b]pyridin-6-yl]-[(3R)-1-(6-methylpyrimidin-4-yl) pyrrolidin-3-yl]methanon;
(3-Chlor-2,4-dimethyl-5,7-dihydropyrrolo[3,4-b]pyridin-6-yl)-[(3R)-1-[6-(difluormethyl)pyrimidin-4-yl]pyrrolidin-3-yl]methanon;
[3-Chlor-2-(methoxymethyl)-4-methyl-5,7-dihydropyrrolo[3,4-b]pyridin-6-yl]-[(3R)-1-pyrimidin-4-ylpyrrolidin-3-yl]methanon;
[3-Chlor-2-(methoxymethyl)-4-methyl-5,7-dihydropyrrolo[3,4-b]pyridin-6-yl]-[(3R)-1-(3-pyridyl)pyrrolidin-3-yl] methanon;
[3-Chlor-2-(methoxymethyl)-4-methyl-5,7-dihydropyrrolo[3,4-b]pyridin-6-yl]-[(3R)-1-[2-(difluormethyl)-4-pyridyl]pyrrolidin-3-yl]methanon;
[3-Chlor-2-(methoxymethyl)-4-methyl-5,7-dihydropyrrolo[3,4-b]pyridin-6-yl]-[(3R)-1-pyrimidin-5-ylpyrrolidin-3-yl]methanon und
[3-Chlor-2-(methoxymethyl)-4-methyl-5,7-dihydropyrrolo[3,4-b]pyridin-6-yl]-[(3R)-1-pyridazin-4-ylpyrrolidin-3-yl]methanon.

**9.** Verbindung der Formel (I) nach Anspruch 1 oder ein pharmazeutisch unbedenkliches Salz davon, ausgewählt aus:

(3-Chlor-2,4-dimethyl-5,7-dihydropyrrolo[3,4-b]pyridin-6-yl)-[(3R)-1-(3-pyridyl)pyrrolidin-3-yl]methanon;
(3-Chlor-2,4-dimethyl-5,7-dihydropyrrolo[3,4-b]pyridin-6-yl)-[(3R)-1-pyrimidin-4-ylpyrroli-din-3-yl]methanon;
(3-Chlor-2,4-dimethyl-5,7-dihydropyrrolo[3,4-b]pyridin-6-yl)-[(3R)-1-[5-(difluormethoxy)-3-pyridyl]pyrrolidin-3-yl]methanon;
(3-Chlor-2,4-dimethyl-5,7-dihydropyrrolo[3,4-b]pyridin-6-yl)-[(3R)-1-[2-(difluormethyl)-4-pyridyl]pyrrolidin-3-yl] methanon;
(3-Chlor-2,4-dimethyl-5,7-dihydropyrrolo[3,4-b]pyridin-6-yl)-[(3R)-1-[2-(trifluormethyl)-4-pyridyl]pyrrolidin-3-yl] methanon;
4-[(3R)-3-(3-Chlor-2,4-dimethyl-5,7-dihydropyrrolo[3,4-b]pyridin-6-carbonyl)pyrrolidin-1-yl ]pyrimidin-2-carbonitril;
(3-Chlor-2,4-dimethyl-5,7-dihydropyrrolo[3,4-b]pyridin-6-yl)-[(3R)-1-pyrimidin-5-ylpyrroli-din-3-yl]methanon;
(3-Chlor-2,4-dimethyl-5,7-dihydropyrrolo[3,4-b]pyridin-6-yl)-[(3R)-1-[6-(difluormethyl)pyrimidin-4-yl]pyrrolidin-3-yl]methanon;
3-Chlor-2-(methoxymethyl)-4-methyl-5,7-dihydropyrrolo[3,4-b]pyridin-6-yl]-[(3R)-1-pyrimidin-4-ylpyrrolidin-3-yl]methanon und
[3-Chlor-2-(methoxymethyl)-4-methyl-5,7-dihydropyrrolo[3,4-b]pyridin-6-yl]-[(3R)-1-(3-pyridyl)pyrrolidin-3-yl] methanon.

**10.** Verfahren zur Herstellung einer Verbindung der Formel (I) nach einem der Ansprüche 1 bis 9 oder eines pharmazeutisch unbedenklichen Salzes davon, umfassend das Reagieren eines Aminsalzes 1

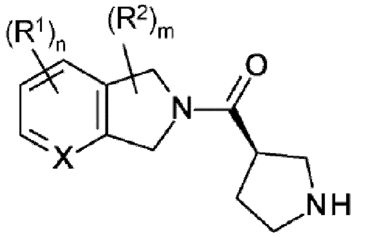

. 2HCl 1

mit einer Halogenidverbindung 2

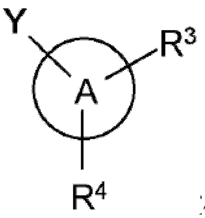

2,

wobei $R^1$, $R^2$, $R^3$, $R^4$, X und A wie in einem der Ansprüche 1 bis 9 definiert sind und Y ein Halogen ist, unter Bildung der Verbindung der Formel (I) und gegebenenfalls Umwandeln der erhaltenen Verbindungen in ein pharmazeutisch unbedenkliches Salz davon.

**11.** Verbindung der Formel (I) nach einem der Ansprüche 1 bis 9 oder ein pharmazeutisch unbedenkliches Salz davon zur Verwendung als therapeutisch wirksame Substanz.

**12.** Pharmazeutische Zusammensetzung, umfassend eine Verbindung der Formel (I) nach einem der Ansprüche 1 bis 9 oder ein pharmazeutisch unbedenkliches Salz davon und einen pharmazeutisch unbedenklichen Hilfsstoff.

**13.** Verbindung der Formel (I) nach einem der Ansprüche 1 bis 9 oder ein pharmazeutisch unbedenkliches Salz davon zur Verwendung bei der therapeutischen und/oder prophylaktischen Behandlung der Alzheimer-Krankheit, Schizophrenie, Psychose, Parkinson-Krankheit, Schmerz, Sucht und Huntington-Krankheit.

## Revendications

**1.** Composé de formule (I)

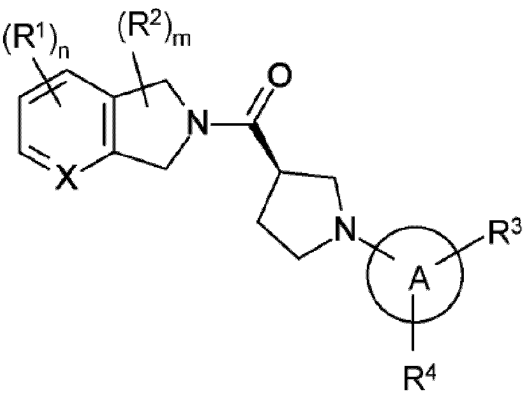

(I)

ou un sel pharmaceutiquement acceptable de celui-ci, dans lequel :

A représente un groupe hétéroaryle à 6 chaînons choisi parmi un pyridinyle, un pyradizinyle, un pyrazinyle et un

pyrimidinyle ;
$R^1$ représente un halogène, un alkyle en $C_1$-$C_6$ ou un alcoxyalkyle en $C_2$-$C_8$ ;
$R^3$ et $R^4$ sont chacun indépendamment choisis parmi un hydrogène, un alkyle en $C_1$-$C_6$, un halogénoalkyle en $C_1$-$C_6$, un alcoxy en $C_1$-$C_6$, un halogénoalcoxy en $C_1$-$C_6$, un cyano, un cycloalkyle en $C_3$-$C_{12}$, un hétérocycloalkyle en $C_2$-$C_9$, $-NR^5R^6$ et $-S(O)_2-R^7$ ;
$R^5$ et $R^6$ sont chacun indépendamment choisis parmi un hydrogène et un alkyle en $C_1$-$C_6$, et $R^7$ représente un alkyle en $C_1$-$C_6$ ;
X représente N ;
n représente 3 ; et
m représente 0.

**2.** Composé de formule (I) selon la revendication 1, ou un sel pharmaceutiquement acceptable de celui-ci, dans lequel A représente un pyridinyle ou un pyrimidinyle.

**3.** Composé de formule (I) selon l'une quelconque des revendications 1 ou 2, ou un sel pharmaceutiquement acceptable de celui-ci, dans lequel $R^1$ représente un chlore, un méthyle ou un méthoxyméthyle.

**4.** Composé de formule (I) selon l'une quelconque des revendications 1 à 3, ou un sel pharmaceutiquement acceptable de celui-ci, dans lequel $R^3$ et $R^4$ sont chacun indépendamment choisis parmi un hydrogène, un halogénoalkyle en $C_1$-$C_6$, un halogénoalcoxy en $C_1$-$C_6$ et un cyano.

**5.** Composé de formule (I) selon l'une quelconque des revendications 1 à 4, ou un sel pharmaceutiquement acceptable de celui-ci, dans lequel $R^3$ et $R^4$ sont chacun indépendamment choisis parmi un hydrogène, $-CHF_2$, $-CF_3$, $-OCHF_2$ et un cyano.

**6.** Composé de formule (I) selon la revendication 1, ou un sel pharmaceutiquement acceptable de celui-ci, dans lequel :

A représente un pyridinyle ou un pyrimidinyle ;
$R^1$ représente un halogène, un alkyle en $C_1$-$C_6$ ou un alcoxyalkyle en $C_2$-$C_8$ ;
$R^3$ et $R^4$ sont chacun indépendamment choisis parmi un hydrogène, un halogénoalkyle en $C_1$-$C_6$, un halogénoalcoxy en $C_1$-$C_6$ et un cyano ;
X représente N ;
n représente 3 ; et
m représente 0.

**7.** Composé de formule (I) selon la revendication 1, ou un sel pharmaceutiquement acceptable de celui-ci, dans lequel :

A représente un pyridinyle ou un pyrimidinyle ;
$R^1$ représente un chlore, un méthyle ou un méthoxyméthyle ;
$R^3$ et $R^4$ sont chacun indépendamment choisis parmi un hydrogène, $-CHF_2$, $-CF_3$, $-OCHF_2$ et un cyano ;
X représente N ;
n représente 3 ; et
m représente 0.

**8.** Composé de formule (I) selon la revendication 1, ou un sel pharmaceutiquement acceptable de celui-ci, choisi parmi :

la (3-chloro-2,4-diméthyl-5,7-dihydropyrrolo[3,4-b]pyridin-6-yl)-[(3R)-1-(3-pyridyl)pyrrolidin-3-yl]méthanone ;
la (3-chloro-2,4-diméthyl-5,7-dihydropyrrolo[3,4-b]pyridin-6-yl)-[(3R)-1-pyrimidin-4-ylpyrrolidin-3-yl]méthanone ;
la (3-chloro-2,4-diméthyl-5,7-dihydropyrrolo[3,4-b]pyridin-6-yl)-[(3R)-1-(4-pyridyl)pyrrolidin-3-yl]méthanone ;
la (3-chloro-2,4-diméthyl-5,7-dihydropyrrolo[3,4-b]pyridin-6-yl)-[(3R)-1-(6-méthyl-3-pyridyl)pyrrolidin-3-yl]méthanone ;
la (3-chloro-2,4-diméthyl-5,7-dihydropyrrolo[3,4-b]pyridin-6-yl)-[(3R)-1-(2-méthyl-4-pyridyl)pyrrolidin-3-yl]méthanone ;
la (3-chloro-2,4-diméthyl-5,7-dihydropyrrolo[3,4-b]pyridin-6-yl)-[(3R)-1-(2-cyclopropylpyrimidin-4-yl)pyrrolidin-3-yl]méthanone ;
la (3-chloro-2,4-diméthyl-5,7-dihydropyrrolo[3,4-b]pyridin-6-yl)-[(3R)-1-[5-(trifluorométhyl)pyrimidin-2-yl]pyrrolidin-3-yl]méthanone ;

la (3-chloro-2,4-diméthyl-5,7-dihydropyrrolo[3,4-b]pyridin-6-yl)-[(3R)-1-(5-méthylpyrimidin-2-yl)pyrrlidin-3-yl]méthanone ;
la (3-chloro-2,4-diméthyl-5,7-dihydropyrrolo[3,4-b]pyridin-6-yl)-[(3R)-1-[6-(trifluorométhyl)-3-pyridyl]pyrrolidin-3-yl]méthanone ;
la (3-chloro-2,4-diméthyl-5,7-dihydropyrrolo[3,4-b]pyridin-6-yl)-[(3R)-1-(6-méthoxy-3-pyridyl)pyrrolidin-3-yl]méthanone ;
la (3-chloro-2,4-diméthyl-5,7-dihydropyrrolo[3,4-b]pyridin-6-yl)-[(3R)-1-[6-(méthylamino)-3-pyridyl]pyrrolidin-3-yl]méthanone ;
la (3-chloro-2,4-diméthyl-5,7-dihydropyrrolo[3,4-b]pyridin-6-yl)-[(3R)-1-(2-méthyl-3-pyridyl)pyrrolidin-3-yl]méthanone ;
la (3-chloro-2,4-diméthyl-5,7-dihydropyrrolo[3,4-b]pyridin-6-yl)-[(3R)-1-(4-méthyl-3-pyridyl)pyrrolidin-3-yl]méthanone ;
la (3-chloro-2,4-diméthyl-5,7-dihydropyrrolo[3,4-b]pyridin-6-yl)-[(3R)-1-[5-(trifluorométhyl)-3-pyridyl]pyrrolidin-3-yl]méthanone ;
la (3-chloro-2,4-diméthyl-5,7-dihydropyrrolo[3,4-b]pyridin-6-yl)-[(3R)-1-[5-(difluorométhyl)-3-pyridyl]pyrrolidin-3-yl]méthanone ;
la (3-chloro-2,4-diméthyl-5,7-dihydropyrrolo[3,4-b]pyridin-6-yl)-[(3R)-1-(5-cyclopropyl-3-pyridyl)pyrrolidin-3-yl]méthanone ;
la (3-chloro-2,4-diméthyl-5,7-dihydropyrrolo[3,4-b]pyridin-6-yl)-[(3R)-1-[5-(difluorométhoxy)-3-pyridyl]pyrrolidin-3-yl]méthanone ;
la (3-chloro-2,4-diméthyl-5,7-dihydropyrrolo[3,4-b]pyridin-6-yl)-[(3R)-1-(5-méthoxy-3-pyridyl)pyrrolidin-3-yl]méthanone ;
la (3-chloro-2,4-diméthyl-5,7-dihydropyrrolo[3,4-b]pyridin-6-yl)-[(3R)-1-(5-méthylsulfonyl-3-pyridyl)pyrrolidin-3-yl]méthanone ;
la (3-chloro-2,4-diméthyl-5,7-dihydropyrrolo[3,4-b]pyridin-6-yl)-[(3R)-1-[2-(difluorométhyl)-4-pyridyl]pyrrolidin-3-yl]méthanone ;
la (3-chloro-2,4-diméthyl-5,7-dihydropyrrolo[3,4-b]pyridin-6-yl)-[(3R)-1-[2-(trifluorométhyl)-4-pyridyl]pyrrolidin-3-yl]méthanone ;
la (3-chloro-2,4-diméthyl-5,7-dihydropyrrolo[3,4-b]pyridin-6-yl)-[(3R)-1-(2-méthoxy-4-pyridyl)pyrrolidin-3-yl]méthanone ;
la (3-chloro-2,4-diméthyl-5,7-dihydropyrrolo[3,4-b]pyridin-6-yl)-[(3R)-1-[2-(trifluorométhyl)pyrimidin-4-yl]pyrrolidin-3-yl]méthanone ;
le 4-[(3R)-3-(3-chloro-2,4-diméthyl-5,7-dihydropyrrolo[3,4-b]pyridine-6-carbonyl)pyrrolidin-1-yl]pyrimidine-2-carbonitrile ;
la (3-chloro-2,4-diméthyl-5,7-dihydropyrrolo[3,4-b]pyridin-6-yl)-[(3R)-1-(2-méthoxypyrimidin-4-yl)pyrrolidin-3-yl]méthanone ;
la (3-chloro-2,4-diméthyl-5,7-dihydropyrrolo[3,4-b]pyridin-6-yl)-[(3R)-1-(6-méthylpyrimidin-4-yl)pyrrolidin-3-yl]méthanone ;
la (3-chloro-2,4-diméthyl-5,7-dihydropyrrolo[3,4-b]pyridin-6-yl)-[(3R)-1-[6-(trifluorométhyl)pyrimidin-4-yl]pyrrolidin-3-yl]méthanone ;
la (3-chloro-2,4-diméthyl-5,7-dihydropyrrolo[3,4-b]pyridin-6-yl)-[(3R)-1-(6-cyclopropylpyrimidin-4-yl)pyrrolidin-3-yl]méthanone ;
la (3-chloro-2,4-diméthyl-5,7-dihydropyrrolo[3,4-b]pyridin-6-yl)-[(3R)-1-(6-méthoxypyrimidin-4-yl)pyrrolidin-3-yl]méthanone ;
la (3-chloro-2,4-diméthyl-5,7-dihydropyrrolo[3,4-b]pyridin-6-yl)-[(3R)-1-pyrimidin-5-ylpyrrolidin-3-yl]méthanone ;
la (3-chloro-2,4-diméthyl-5,7-dihydropyrrolo[3,4-b]pyridin-6-yl)-[(3R)-1-(2-méthylpyrimidin-5-yl)pyrrolidin-3-yl]méthanone ;
la (3-chloro-2,4-diméthyl-5,7-dihydropyrrolo[3,4-b]pyridin-6-yl)-[(3R)-1-[2-(trifluorométhyl)pyrimidin-5-yl]pyrrolidin-3-yl]méthanone ;
la (3-chloro-2,4-diméthyl-5,7-dihydropyrrolo[3,4-b]pyridin-6-yl)-[(3R)-1-(2-méthoxypyrimidin-5-yl)pyrrolidin-3-yl]méthanone ;
la (3-chloro-2,4-diméthyl-5,7-dihydropyrrolo[3,4-b]pyridin-6-yl)-[(3R)-1-[2-(oxetan-3-yl)pyrimidin-5-yl]pyrrolidin-3-yl]méthanone ;
la (3-chloro-2,4-diméthyl-5,7-dihydropyrrolo[3,4-b]pyridin-6-yl)-[(3R)-1-[2-(méthylamino)pyrimidin-5-yl]pyrrolidin-3-yl]méthanone ;
la (3-chloro-2,4-diméthyl-5,7-dihydropyrrolo[3,4-b]pyridin-6-yl)-[(3R)-1-[2-(diméthylamino)pyrimidin-5-yl]pyrrolidin-3-yl]méthanone ;

la (3-chloro-2,4-diméthyl-5,7-dihydropyrrolo[3,4-b]pyridin-6-yl)-[(3R)-1-(4-méthylpyrimidin-5-yl)pyrrolidin-3-yl]méthanone ;
la (3-chloro-2,4-diméthyl-5,7-dihydropyrrolo[3,4-b]pyridin-6-yl)-[(3R)-1-pyridazin-4-ylpyrrolidin-3-yl]méthanone ;
la (3-chloro-2,4-diméthyl-5,7-dihydropyrrolo[3,4-b]pyridin-6-yl)-[(3R)-1-pyridazin-3-ylpyrrolidin-3-yl]méthanone ;
la (3-chloro-2,4-diméthyl-5,7-dihydropyrrolo[3,4-b]pyridin-6-yl)-[(3R)-1-(6-méthylpyridazin-3-yl)pyrrolidin-3-yl]méthanone ;
la (3-chloro-2,4-diméthyl-5,7-dihydropyrrolo[3,4-b]pyridin-6-yl)-[(3R)-1-[6-(trifluorométhyl)pyridazin-3-yl]pyrrolidin-3-yl]méthanone ;
la (3-chloro-2,4-diméthyl-5,7-dihydropyrrolo[3,4-b]pyridin-6-yl)-[(3R)-1-(6-méthoxypyrazin-2-yl)pyrrolidin-3-yl]méthanone ;
la (3-chloro-2,4-diméthyl-5,7-dihydropyrrolo[3,4-b]pyridin-6-yl)-[(3R)-1-(6-cyclopropylpyrazin-2-yl)pyrrolidin-3-yl]méthanone ;
le 5-[(3R)-3-(3-chloro-2,4-diméthyl-5,7-dihydropyrrolo[3,4-b]pyridine-6-carbonyl)pyrrolidin-1-yl]pyrazine-2-carbonitrile ;
la (3-chloro-2,4-diméthyl-5,7-dihydropyrrolo[3,4-b]pyridin-6-yl)-[(3R)-1-(5-méthoxypyrazin-2-yl)pyrrolidin-3-yl]méthanone ;
la (3-chloro-2,4-diméthyl-5,7-dihydropyrrolo[3,4-b]pyridin-6-yl)-[(3R)-1-(5-méthylpyrazin-2-yl)pyrrolidin-3-yl]méthanone
la (3-chloro-2,4-diméthyl-5,7-dihydropyrrolo[3,4-b]pyridin-6-yl)-[(3R)-1-[5-(trifluorométhyl)pyrazin-2-yl]pyrrolidin-3-yl]méthanone ;
la (3-chloro-2,4-diméthyl-5,7-dihydropyrrolo[3,4-b]pyridin-6-yl)-[(3R)-1-(4-méthoxypyrimidin-2-yl)pyrrolidin-3-yl]méthanone ;
la (3-chloro-2,4-diméthyl-5,7-dihydropyrrolo[3,4-b]pyridin-6-yl)-[(3R)-1-(4-méthylpyrimidin-2-yl)pyrrolidin-3-yl]méthanone ;
la (3-chloro-2,4-diméthyl-5,7-dihydropyrrolo[3,4-b]pyridin-6-yl)-[(3R)-1-[4-(trifluorométhyl)pyrimidin-2-yl]pyrrolidin-3-yl]méthanone ;
la (3-chloro-2,4-diméthyl-5,7-dihydropyrrolo[3,4-b]pyridin-6-yl)-[(3R)-1-(6-méthylpyrazin-2-yl)pyrrolidin-3-yl]méthanone ;
la (3-chloro-2,4-diméthyl-5,7-dihydropyrrolo[3,4-b]pyridin-6-yl)-[(3R)-1-(6-méthylpyridazin-4-yl)pyrrolidin-3-yl]méthanone ;
la [3-chloro-2-(méthoxyméthyl)-4-méthyl-5,7-dihydropyrrolo[3,4-b]pyridin-6-yl]-[(3R)-1-(6-méthylpyrimidin-4-yl)pyrrolidin-3-yl]méthanone ;
la (3-chloro-2,4-diméthyl-5,7-dihydropyrrolo[3,4-b]pyridin-6-yl)-[(3R)-1-[6-(difluorométhyl)pyrimidin-4-yl]pyrrolidin-3-yl]méthanone ;
la [3-chloro-2-(méthoxyméthyl)-4-méthyl-5,7-dihydropyrrolo[3,4-b]pyridin-6-yl]-[(3R)-1-pyrimidin-4-ylpyrrolidin-3-yl]méthanone ;
la [3-chloro-2-(méthoxyméthyl)-4-méthyl-5,7-dihydropyrrolo[3,4-b]pyridin-6-yl]-[(3R)-1-(3-pyridyl)pyrrolidin-3-yl]méthanone ;
la [3-chloro-2-(méthoxyméthyl)-4-méthyl-5,7-dihydropyrrolo[3,4-b]pyridin-6-yl]-[(3R)-1-[2-(difluorométhyl)-4-pyridyl]pyrrolidin-3-yl]méthanone ;
la [3-chloro-2-(méthoxyméthyl)-4-méthyl-5,7-dihydropyrrolo[3,4-b]pyridin-6-yl]-[(3R)-1-pyrimidin-5-ylpyrrolidin-3-yl]méthanone ; et
la [3-chloro-2-(méthoxyméthyl)-4-méthyl-5,7-dihydropyrrolo[3,4-b]pyridin-6-yl]-[(3R)-1-pyridazin-4-ylpyrrolidin-3-yl]méthanone.

**9.** Composé de formule (I) selon la revendication 1, ou un sel pharmaceutiquement acceptable de celui-ci, choisi parmi :

la (3-chloro-2,4-diméthyl-5,7-dihydropyrrolo[3,4-b]pyridin-6-yl)-[(3R)-1-(3-pyridyl)pyrrolidin-3-yl]méthanone ;
la (3-chloro-2,4-diméthyl-5,7-dihydropyrrolo[3,4-b]pyridin-6-yl)-[(3R)-1-pyrimidin-4-ylpyrrolidin-3-yl]méthanone ;
la (3-chloro-2,4-diméthyl-5,7-dihydropyrrolo[3,4-b]pyridin-6-yl)-[(3R)-1-[5-(difluorométhoxy)-3-pyridyl]pyrrolidin-3-yl]méthanone ;
la (3-chloro-2,4-diméthyl-5,7-dihydropyrrolo[3,4-b]pyridin-6-yl)-[(3R)-1-[2-(difluorométhyl)-4-pyridyl]pyrrolidin-3-yl]méthanone ;
la (3-chloro-2,4-diméthyl-5,7-dihydropyrrolo[3,4-b]pyridin-6-yl)-[(3R)-1-[2-(trifluorométhyl)-4-pyridyl]pyrrolidin-3-yl]méthanone ;

le 4-[(3R)-3-(3-chloro-2,4-diméthyl-5,7-dihydropyrrolo[3,4-b]pyridine-6-carbonyl)pyrrolidin-1-yl]pyrimidine-2-carbonitrile ;
la (3-chloro-2,4-diméthyl-5,7-dihydropyrrolo[3,4-b]pyridin-6-yl)-[(3R)-1-pyrimidin-5-ylpyrrolidin-3-yl]méthanone ;
la (3-chloro-2,4-diméthyl-5,7-dihydropyrrolo[3,4-b]pyridin-6-yl)-[(3R)-1-[6-(difluorométhyl)pyrimidin-4-yl]pyrrolidin-3-yl]méthanone ;
la 3-chloro-2-(méthoxyméthyl)-4-méthyl-5,7-dihydropyrrolo[3,4-b]pyridin-6-yl]-[(3R)-1-pyrimidin-4-ylpyrrolidin-3-yl]méthanone ; et
la [3-chloro-2-(méthoxyméthyl)-4-méthyl-5,7-dihydropyrrolo[3,4-b]pyridin-6-yl]-[(3R)-1-(3-pyridyl)pyrrolidin-3-yl]méthanone.

**10.** Procédé pour la préparation d'un composé de formule (I) selon l'une quelconque des revendications 1 à 9, ou d'un sel pharmaceutiquement acceptable de celui-ci, comprenant la réaction d'un sel d'amine 1

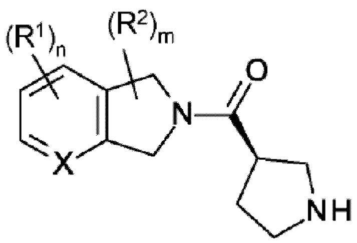

. 2HCl **1**

avec un composé d'halogénure 2

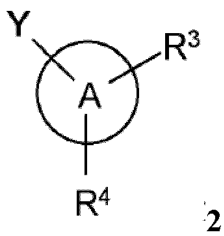

**2**

dans lequel $R^1$, $R^2$, $R^3$, $R^4$, X et A sont tels que définis dans l'une quelconque des revendications 1 à 9 et Y représente un halogène, pour former ledit composé de formule (I), et éventuellement la conversion des composés obtenus en un sel pharmaceutiquement acceptable de ceux-ci.

**11.** Composé de formule (I) selon l'une quelconque des revendications 1 à 9, ou un sel pharmaceutiquement acceptable de celui-ci, pour une utilisation comme substance thérapeutiquement active.

**12.** Composition pharmaceutique comprenant un composé de formule (I) selon l'une quelconque des revendications 1 à 9, ou un sel pharmaceutiquement acceptable de celui-ci, et un véhicule pharmaceutiquement acceptable.

**13.** Composé de formule (I) selon l'une quelconque des revendications 1 à 9, ou un sel pharmaceutiquement acceptable de celui-ci, pour une utilisation dans le traitement thérapeutique et/ou prophylactique de la maladie d'Alzheimer, de la schizophrénie, de la psychose, de la maladie de Parkinson, de la douleur, de l'addiction et de la maladie de Huntington.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018069732 A1
- WO 2018002760 A


### Non-patent literature cited in the description


- **LEBOIS et al.** *Neuropharmacology*, 2018, vol. 136, 362-373
- **PARDIÑAS et al.** *Nature genetics*, 2018, vol. 50, 381-389
- **BYUN et al.** *Neuropsychopharmacology*, 2014, vol. 39, 1578
- **THORN et al.** *Hippocampus*, 2017, vol. 27, 794-810
- **BUBSER et al.** *ACS Chemical Neuroscience*, 2014, vol. 5, 920-942
- **BODICK et al.** *Arch Neurol*, 1997, vol. 54, 465-73
- **TZAVARA et al.** *The FASEB Journal*, 2004, vol. 18, 1410-1412
- **SCHMIDT et al.** *Psychopharmacology*, 2011, vol. 216, 367-378
- **DENCKER et al.** *Psychopharmacology*, 2012, vol. 224, 277-287
- **PANCANI et al.** *Proceedings of the National Academy of Sciences*, 2015, vol. 112, 14078-14083
- **SHEN et al.** *Neuron*, 2015, vol. 88, 762-773
- **SCHECHTMANN et al.** *Pain*, 2008, vol. 139, 136-145
- **CAI et al.** *Journal of Neurochemistry*, 2009, vol. 111, 1000-1010
- **T. W. GREENE** ; **P. G. M. WUTTS**. Protective Groups in Organic Chemistry. John Wiley & Sons, 2014
- **T. W. GREENE** ; **P. G. M. WUTS**. Protective Groups in Organic Chemistry. Wiley, 1999
- **RICHARD C. LAROCK.** Comprehensive Organic Transformations: A Guide to Functional Group Preparations. John Wiley & Sons, 1999